(19) Europäisches Patentamt
European Patent Office
Office européen des brevets

(11) Publication number: **0 136 879**
**A2**

(12) # EUROPEAN PATENT APPLICATION

(21) Application number: **84306556.6**

(22) Date of filing: **26.09.84**

(51) Int. Cl.$^4$: **C 07 K 5/02**
C 07 K 5/06, C 07 C 103/50
C 07 C 103/48, C 07 C 103/46
C 07 C 131/00
//C07C69/708, C07C125/065, C07C101/18, C07C99/00

(30) Priority: **30.09.83 JP 182000/83**
**24.07.84 JP 153430/84**

(43) Date of publication of application:
**10.04.85 Bulletin 85/15**

(84) Designated Contracting States:
**AT BE CH DE FR GB IT LI NL SE**

(71) Applicant: **YAMANOUCHI PHARMACEUTICAL CO. LTD.**
**No. 5-1 Nihonbashi-Honcho, 2-chome Chuo-ku**
**Tokyo(JP)**

(72) Inventor: **Tsukamoto, Shin-Ichi**
**No. 1-39-14, Sakashita Itabashi-ku**
**Tokyo(JP)**

(72) Inventor: **Nagano, Yoshinobu**
**No. 2-15-14, Midori-cho**
**Tanashi-shi Tokyo(JP)**

(72) Inventor: **Katsuda, Kimio**
**No. 1501, Kubota Yoshima-cho**
**Hiki-gun Saitama(JP)**

(74) Representative: **Geering, Keith Edwin et al,**
**REDDIE & GROSE 16 Theobalds Road**
**London WC1X 8PL(GB)**

(54) **Fatty acid derivatives and processes of producing them.**

(57) Water soluble fibrinolytic fatty acid derivatives of formula (I), and salts thereof

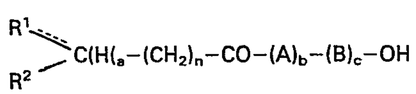

(I)

in which

$R^1$ represents an alkanoylamino group the carbon chain of which may be interrupted by at least one oxygen atom, an alkanoyloxy group the carbon chain of which is interrupted by at least one oxygen atom, an alkoxyimino group, or a group shown by the formula $R^3-(NH-Y-CO)_m-Z-$(wherein

$R^3$ represents an alkanoyl group or a $C_1-C_5$ alkylsulfonyl group; Y represents an alkylene group; m represents 1 or 2; and Z represents an oxygen atom or an imino group);

$R^2$ represents an alkyl group, an alkylaminocarbonyl group the carbon chain of which may be interrupted by at least one oxygen atom, or an alkoxyaminocarbonyl group the carbon chain of which is interrupted by at least one oxygen atom;

$\overset{\cdots}{=}$ represents a single bond or a double bond;

a is zero when $\overset{\cdots}{=}$ is a double bond and 1 when $\overset{\cdots}{=}$ is a single bond;

b is 0 or 1; c is 0 or 1;

n represents an integer of 1 to 3; and A and B are the same or different amino acid residues.

Various processes for the production of these compounds are as described.

EP 0 136 879 A2

Croydon Printing Company Ltd.

# FATTY ACID DERIVATIVES AND PROCESSES OF PRODUCING THEM

This invention relates to fatty acid derivatives and salts thereof showing fibrinolytic action and water solubility. The invention further relates to processes of producing these fatty acid derivatives and salts thereof.

Various methods for prophylaxis or treatment of thrombosis by dissolving the fibrin clot of arteriovenous thrombosis have been proposed and investigated, e.g. administering plasmin, administering plasminogen activator, releasing plasminogen activator by the administration of a medicament, restraining a plasmin inhibitor, etc., but since these methods all have difficulties Urokinase is still the only fibrinolytic agent on the market.

However, Urokinase has the problems that it cannot be orally administered and must be administered at a high dosage, so that its use is costly, and hence the development of other compounds which can be administered orally and at low cost has been desired.

Hitherto, low molecular fibrinolytic agents which can be orally administered have been investigated but no such fibrinolytic agents have yet been practically used.

Recently, as compounds possessing fibrinolytic action, acylpeptides shown by following general formulae (A) and (B) were proposed (European Patent Publication (Unexamined) Nos. 54,435 and 85,255);

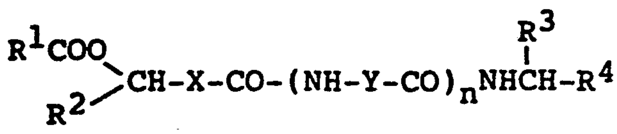

(A)

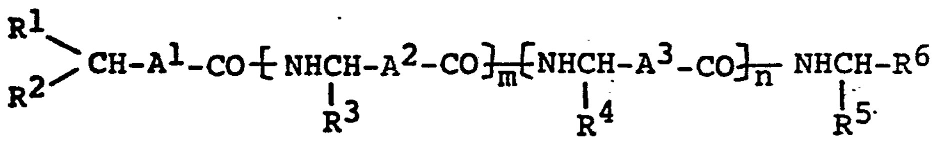

(B)

However, for clinical use as fibrinolytic agents, the foregoing compounds are still insufficient in fibrinolytic activity, and almost all the foregoing known compounds show a low solubility in water, making it difficult to attain sufficient phamacoligical activity and to form preparations of these compounds.

As the result of synthesizing various novel fibrinolytic compounds and investigating the fibrinolytic activity of these compounds, it has been discovered that the compounds shown by following general formula (I) and the pharmaceutically acceptable salts of them are very

excellent in fibrinolytic activity and show a high solubility in water, and based on the discovery, the invention has been accomplished.

According to this invention there is provided a fatty acid derivative represented by following general formula (I) or a salt thereof;

$$\begin{matrix} R^1 \\ R^2 \end{matrix} \!\!> C(H)_a-(CH_2)_n-CO-(A)_b-(B)_c-OH \qquad (I)$$

in which

$R^1$ represents an alkanoylamino group the carbon chain of which may be interrupted by at least one oxygen atom, an alkanoyloxy group the carbon chain of which is interrupted by at least one oxygen atom, an alkoxyimino group, or a group shown by $R^3-(NH-Y-CO)_m-Z-$ (wherein $R^3$ represents an alkanoyl group or a $C_1-C_5$ alkylsulfonyl group; Y represents an alkylene group; m is 1 or 2; and Z represents an oxygen atom or an imino group);

$R^2$ represents an alkyl group, an alkylaminocarbonyl group the carbon chain of which may be interrupted by at least one oxygen atom, or an alkoxyaminocarbonyl group the carbon chain of which is interrupted by at least one oxygen atom;

- - - - represents a single bond or a double bond;

a is zero when - - - - is a double bond and 1 and when - - - - is a single bond;

b is 0 or 1; c is 0 or 1;

n represents an integer of 1 to 3; and

A and B are same or different amino acid residues.

In the definitions of the groups used for the indications of the general formulae in this specification, the term "lower" means a straight or branched carbon chain having 1 to 5 carbon atoms, unless otherwise indicated.

Accordingly, a lower alkyl group or moiety (e.g. in a lower alkylsulfonyl group) includes a methyl group, an ethyl group, a propyl group, an isopropyl group, a butyl group, an isobutyl group, a sec-butyl group, a tert-butyl group, a pentyl (or n-amyl) group, an isopentyl group, a tert-pentyl group, and a neopentyl group.

Also, in the definitions of the groups of the general formulae in this specification, the term "the carbon chain may be (or is) interrupted by at least one oxygen atom" can mean an ether-type or polyether-type carbon chain in which 1 to 4 oxygen atoms may be (or are) present at an optional position or positions in the chain; a preferred example of such a group is shown by the formula

$$R^{10} - (O - Y^1)_p - O - Y^2 - (D)_d -$$

wherein $R^{10}$ represents an alkyl group; $Y^1$ represents an alkylene group having 2 to 6 carbon atoms; p represents

0 or an integer of 1 to 3; $X^2$ represents a single bond or an alkylene group having 1 to 6 carbon atoms; d is 0 or 1 and D represents a carbamide.

group (-CONH-), a carbamoyl group (-NHCO-), or a carbonyloxy group (-COO-).

The alkyl group or the alkyl moiety of the alkylaminocarbonyl group in this invention is preferably selected from straight or branched groups having 1 to 20 carbon atoms and specific examples are a hexyl group, an isohexyl group, a heptyl group, an isoheptyl group, an octyl group, an isooctyl group, a nonyl group, an isononyl group, a decyl group, an isodecyl group, a undecyl group, an isoundecyl group, a dodecyl group, an isododecyl group, a tridecyl group, an isotridecyl group, a tetradecyl group, an isotetradecyl group, a pentadecyl group, an isopentadecyl group, a hexadecyl group, an isohexadecyl group, a heptadecyl group, an isoheptadecyl group, an octadecyl group, an isooctadecyl group, a nonadecyl group, an isononadecyl group, an eicosyl group, an isoeicosyl group, in addition to the lower alkyl groups described hereinbefore.

The alkylene group Y above is preferably selected from groups having 1 to 6 carbon atoms and specific examples are a methylene group, an ethylene group, a trimethylene group, a propylene group ($-CH(CH_3)CH_2-$ or $-CH_2CH(CH_3)-$), a tetramethylene group, a pentamethylene group, and a hexamethylene group; the alkylene group $Y^1$ above preferably has 2 to 5 carbon atoms.

The alkanoyl group and the alkanoyl moiety of

the alkanoylamino group and the alkanoyloxy group in this invention are preferably selected from those of 2 to 20 carbon atoms and specific examples are an acetyl group, a propionyl group, a butyryl group, an isobutyryl group, a valeryl group, an isovaleryl group, a pivaloyl group, a hexanoyl group, a heptanoyl group, an octanoyl group, a nonanoyl group, a decanoyl group, an undecanoyl group, a lauroyl group, a tridecanoyl group, a myristoyl group, a pentadecanoyl group, a palmitoyl group, a heptadecanoyl group, a stearoyl group, a nonadecanoyl group, an eicosanoyl group.

The alkoxy moiety of the alkoxyimino group and the alkoxyaminocarbonyl group in this invention is preferably selected from those of 1 to 20 carbon atoms and specific examples : a methoxy group, an ethoxy group, a propoxy group, an isopropoxy group, a butoxy group, an isobutoxy group, a pentyloxy group, an isopentyloxy group, a hexyloxy group, an isohexyloxy group, a heptyloxy group, an isoheptyloxy group, an octyloxy group, an isooctyloxy group, a nonyloxy group, an isononyloxy group, a decyloxy group, an isodecyloxy group, an undecyloxy group, an isoundecyloxy group, a dodecyloxy group, an isododecyloxy group, a tridecyloxy group, an isotridecyloxy group, a tetradecyloxy group, a pentadecyloxy group, a hexadecyloxy group, a heptadecyloxy group, an octadecyloxy group, a nonadecyloxy group, an eicosyloxy group.

Thus any alkanoylamino,

alkanoyloxy, alkylaminocarbonyl or alkoxyaminocarbonyl group the carbon chain of which is interrupted by at least one oxygen atom may be the monoxa-, dioxa, trioxa- or tetraoxa- group

with the 1 to 4 oxygen atoms optionally positioned in the carbon chain, preferably in the position as shown by $R^{10}-(O-Y^1)_p-O-Y^2-D-$ (wherein $Y^1$, $Y^2$, D, d and p are as defined above).

Any amino acid residue in the compounds of this invention is a natural or synthetic amino acid in which the hydrogen atom is removed from the amino nitrogen (N end) and the hdyroxy group is removed from the carboxy group (C end) and is preferably selected from those of the formula

$$\left[ NH - \underset{R^{11}}{\underset{|}{CH}} - Y^3 - CO \right],$$

wherein $R^{11}$ represents a hydrogen atom, a lower alkyl group, a hydroxy lower alkyl group, an amino lower alkyl group, a carboxy lower alkyl group, a guanidino lower alkyl group, a lower alkylthio lower alkyl group, a phenyl lower alkyl group, a hydroxyphenyl lower alkyl group, a hydroxyphenoxyphenyl lower alkyl group, an

imidazolyl lower alkyl group, or a thienyl group, 1 is 0 or 1, and $Y^3$ represents a lower alkylene group.

Examples of the particularly preferred amino acid residue are a serine residue (-Ser-), a phenylalanine residue (-Phe-), a ß-alanine residue (-ß-Ala-), a glutamic acid residue (-Glu-), an aspartic acid residue (-Asp-), a glycine residue (-Gly-), a tyrosine residue (-Tyr-),

Some compounds of this invention

contain an asymmeteric carbon atom in the molecule giving rise to various optical isomers. Thus invention includes each separate isomer and any mixture of isomers.

Also, some of the compounds of this invention form salts which are included in the invention; pharmaceutically acceptable nontoxic salts of the compounds of foregoing general formula (I) include for example salts of alkali metals (such as sodium, potassium) and alkaline earth metals (such as calcium, magnesium); salts of bases such as trimethylamine, triethylamine, cyclohexylamine, dicyclohexylamine, diethanolamine, alginine, lysine, salts of acids such as hydrochloric acid, sulfuric acid, hydrobromic acid, phosphoric acid, formic acid, acetic acid, oxalic acid, succinic acid, fumaric acid, maleic acid, methanesulfonic acid, ethanesulfonic acid, benzenesulfonic acid; and ammonium salts.

Fatty acid derivatives shown by following general formula (I') and salts thereof are preferred compounds of this invention:

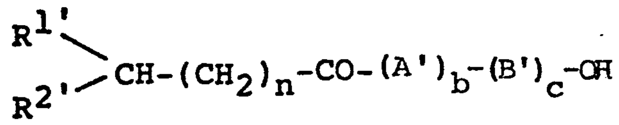 (I')

in which b and c are each 0 or 1;

$R^{1'}$ represents a group $R^{10}-(O-Y^1)_p-O-Y^2-D^1$ (wherein $R^{10}$, $Y^1$, $Y^2$, and p are as defined above, and $D^1$ represents a carbamide group or a carbonyloxy group);

$R^{2'}$ represents an alkyl group having 1 to 20 carbon atoms or a group $R^{10}-(O-Y^1)_p-O-Y^2-D^2-$ (wherein $R^{10}$, $Y^1$, $Y^2$ and p are as defined above and $D^2$ represents a carbamoyl group (-NHCO-));

n represents an integer of 1 to 3; and

A' and B' are the same or different and selected from a serine residue, a phenylalanine residue, a ß-alanine residue, a glutamic acid residue, an aspartic acid residue, a glycine residue, and a tyrosine residue.

Examples of particularly preferred compounds of of Formula I' are as follows:

N-[3-(3,6-dioxahexanoyloxy)octadecanoyl]-L-phenylalanine;

N-[3-(3,6-dioxahexanoyloxy)octadecanoyl]-L-serine;

N-[3-(3,6,9-trioxapentadecanoyloxy)octadecanoyl]-L-phenylalanine;

N-[3-(3,6,9,12-tetraoxahexadecanoyloxy)octadecanoyl]-L-phenylalanine; and

(S)-3-(3,6-dioxahexadecanamido)-3-(3-oxatridecylcarbamoyl)propionic acid.

The compounds shown by above general

formula (I) and the salts thereof can be prepared by the following processes A to F of the invention.

Process A (Release of protective group for the carboxy group):

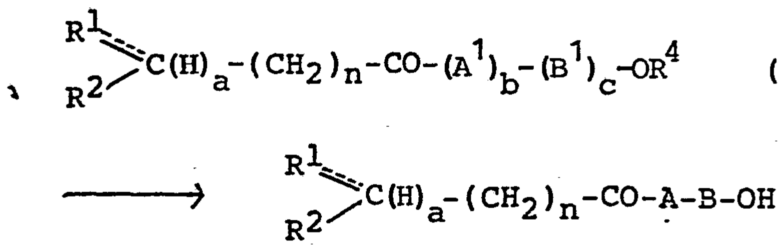

(II)

(I)

wherein $R^1$, $R^2$, ═══, a, b, c, n, A, and B are as defined above; $A^1$ and $B^1$ are the same or different amino acid residues which may be protected; and $R^4$ represents a protective group for the carboxy group.

As shown above, a compound (I) can be produced by release of protective group(s) from the corresponding protected compound (II).

In this case, any protective group for an amino acid residue of $A^1$ or $B^1$, and the protective group $R^4$, may be selected from those which are widely used for such purpose in the field of peptide chemistry and which can be released under mild conditions.

Examples of such a protective group for the amino group are acyl groups such as a t-butoxycarbonyl group, a benzyloxycarbonyl group, a p-methoxybenzyloxycarbonyl group, a cyclohexanecarbonyl group, a phthaloyl group, a trifluoroacetyl group; and aralkyl groups such as a benzhydryl group, a trityl group. Examples of ´ protective group for the carboxy group are ester residues for forming a methyl ester, an ethyl ester, a propyl ester, a butyl ester, a t-butyl ester, an acetoxymethyl ester, a pivaloylmethyl ester, a benzylcarbonyloxymethyl ester, a benzoyloxymethyl ester, a phthalidyl ester, a 5-oxo-2-tetrahydrofuryl ester, a benzyl ester, a p-nitrobenzyl ester, a benzhydryl ester, a trityl ester, a phenyl ester . Also, examples of protective group for the hydroxy group are acyl groups such as an acetyl group, a propionyl group, a butyryl group, a valeryl group; and aralkyl groups such as a benzyl group, a benzhydryl group, a trityl group.

The releasing reaction for an ester residue as the protective group for a carboxy group is suitably selected from the methods widely used in the field of peptide chemistry according to the kind of starting material, particularly the ester residue. As such reactions there are hydrolysis by acid catalyst, saponification under basic conditions, and

catalytic or chemical reduction.

For example, when the ester residue is a tert-butyl group or a benzhydryl group, it is simple and suitable to dissolve the starting material of general formula (II) in a solvent such as trifluoroacetic acid, a mixture of trifluoroacetic acid and anisole, a mixture of hydrobromic acid and acetic acid, or a mixture of hydrochloric acid and dioxane, and treat the solution under cooling or at room temperature.

When the ester residue is a lower alkyl group such as a methyl group or ethyl group, it is suitable to dissolve the starting material of general formula (II) in a solvent such as methanol, or ethanol and saponify it in the presence of a base such as sodium hydroxide, or potassium hydroxide under heating.

When the ester residue is e.g. a benzyl group, a benzhydryl group or a p-nitrobenzyl group, the ester group can be easily released by catalytic reduction using e.g. palladium carbon as catalyst.

When any amino acid residue $A^1$ and/or $B^1$ has a carboxy group substituted by an ester residue, the ester residues may be simultaneously released or a desired ester residue only may be selectively released.

When amino acid residue $A^1$ and/or $B^1$ has a protective group for the amino group or a protective group for the hydroxy group, the release

of the protective group can be performed by a method conventionally used in the field of peptide chemistry. For example, the release of a protective group for an amino group when this is an aralkyl group such as a trityl group or an acyl group as described above can be performed easily by hydrolysis using an acid - preferably, e.g. formic acid, trifluoroacetic acid or hydrochloric acid.

When using, for example, a benzyl group as a protective group for the hydroxy group, catalytic reduction using palladium-carbon as the catalyst can be employed for releasing the protective group.

The release of protective groups for the carboxy group, for the hydroxy group and/or for the amino group can be performed simultaneously.

Process B (Amide-formation process ):

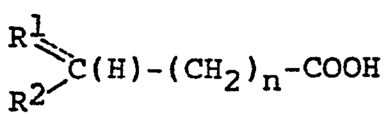 (Ia) or a reactive deriva-

tive thereof
1) $H-(A^2)_b-B^2-OR^5$ (III)
2) Release of protective group(s) if the compound has such protective group(s)

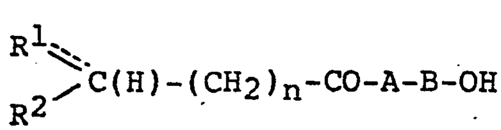 (I)

wherein $R^1$, $R^2$, ----, a, b, c, A, B and n are as defined

above except that A and B are not a single bond at the same time; $R^5$ represents a hydrogen atom or $R^4$ as defined above; and $A^2$ and $B^2$ are amino acid residues which may have one or more protective groups.

Compounds (I) can be produced by reacting carboxylic acid (Ia) or reactive derivative thereof with amino acid or peptide (III).

Suitable reactive derivatives of the compound (Ia) include acid halides such as acid chloride, acid bromide; acid azides; active esters such as those with N-hydroxybenzotriazole or N-hydroxysuccinimide, or the p-nitrophenyl ester; symmetric acid anhydrides; alkyl carbonate mixed acid anhydrides; and mixed acid anhydrides with p-toluenesulfonic acid.

It is advantageous to use compound (Ia) or the reactive derivative thereof with an equimolor amount or a slight molar excess or deficiency of compound (III) in an organic solvent inactive to the reaction, under cooling or at room temperature. The solvent is selected according to the starting material used and may comprise e.g. tetrahydrofuran, dioxane, ether, benzene, toluene, xylene, methylene chloride, dichloroethane, chloroform, carbon tetrachloride, dimethylformamide, ethyl acetate, or acetonitrile.

When compound (Ia) is reacted

in the form of the free carboxylic acid, it is advantageous to perform the reaction in the presence of a condensing agent such as N,N'-dicyclohexylcarbodiimide or 1,1-carbonylimidazole.

It is sometimes advantageous, for smooth operation, to perform the reaction in the presence of tertiary base (such as triethylamine, pyridine, picoline, lutidine, N,N-dimethylaniline) or inorganic base (such as potassium carbonate, sodium carbonate, sodium hydroxide), according to the starting material used.

When active ester is used as reactive derivative of compound (Ia), the amide compound can be obtained even when the carboxy group of compound (III) is not substituted by the ester residue, and thus $R^5$ of compound (III) may be a hydrogen atom.

When the amide compound thus formed has one or more protective groups, these may be released in the same manner as in Process A.

Process C (Amide formation process ):

$$\begin{matrix} H_2N \\ R^2 \end{matrix} > CH-(CH_2)_n-CO-(A^1)_b-(B^1)_c-OR^5 \qquad (IV)$$

1) $R^6$-OH (V) or a reactive derivative thereof

2) release of protective group(s) when the compound is protected.

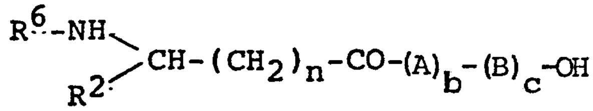

(Ib)

wherein $R^2$, $R^5$, $A^1$, $B^1$, A, B, b, c and n are as defined above and $R^6$ represents an alkanoyl group the carbon chain of which may be interrupted by at least one oxygen atom or a group $R^3-(NH-Y-CO)_m-$ (wherein $R^3$, Y and m are as defined above).

Amide compound (Ib) is produced by reacting amine compound (IV) and carboxylic acid (V) or reactive derivative thereof.

The kind of the solvent in this reaction, the amounts of starting material, and other reaction conditions (such as temperature) may be as in Process B.

Process D (Amide-formation process):

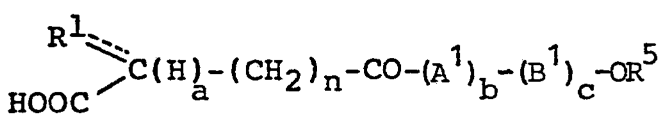

(VI) or

reactive derivative thereof

1) $R^7-NH_2$ (VII)

2) release of protective group(s) when the compound is protected

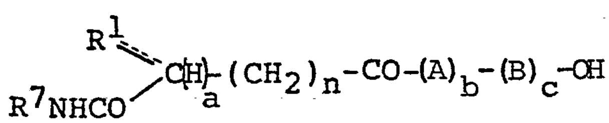

(Ic)

wherein $R^1$, $R^5$, $A^1$, $B^1$, A, B, ═══, a, b, c and n are as defined above and $R^7$ represents an alkyl group the carbon chain of which may be interrupted by at least one oxygen atom or an alkoxy group the carbon chain of which is interrupted by at least one oxygen atom.

Compound (Ic) is produced by reacting carboxylic acid (VI) or reactive derivative thereof and amine (VII).

The reaction conditions may be as in Process B and Process C.

Process E (Esterification):

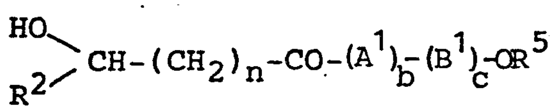 (VIII) or reactive derivative thereof

1) $R^8$-OH (IX) or reactive derivative thereof

2) release of protective group(s) when the compound is protected

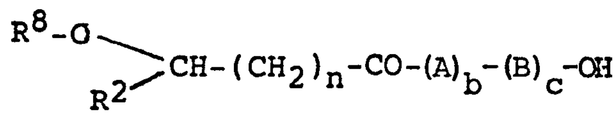 (Id)

wherein $R^2$, $R^5$, $A^1$, $B^1$, A, B, b, c and n are as defined above and $R^8$ represents an alkanoyl group the carbon chain of which is interrupted by at least one oxygen atom or a group $R^3-(NH-Y-CO)_m-$ (wherein $R^3$, Y and m are as defined above.

Compound (Id) is produced by reacting alcohol (VIII) or reactive derivative thereof and carboxylic acid (IX) or reactive derivative thereof.

The reaction is a general ester-forming reaction and can be performed conventionally. However, reaction in the

presence of condensing agent, e.g. dicyclohexylcarbodiimide, is advantageous. It is suitable to react compound (VIII) and an equimolar or slightly excessive or deficient molar amount of compound (IX) in an organic solvent inactive to the reaction, e.g. as dimethylformamide or methylene chloride, at room temperature or under heating. It is sometimes advantageous for the smooth progress of the reaction to add a base such as dimethylaminopyridine to the reaction system.

The reaction can be performed conventionally using an acid halide or acid anhydride as a reactive derivative of the acid component, or a halogen substitution product or tosylate derivative shown by the general formula

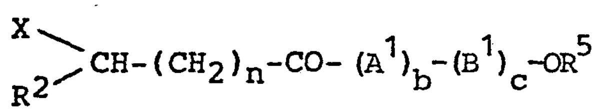

(wherein $R^2$, $R^5$, $A^1$, $B^1$, b, c and n are as above and X is halogen (e.g. Cl or Br) or a toluenesulfonyloxy group), as a reactive derivative of the alcohol component.

Process F (Alkoxyimino-formation ):

$R^2-CO-(CH_2)_n-CO-(A^1)_b-(B^1)_c-OR^4$ (XII)

1) $R^9-O-NH_2$ (XIII)

2) release of protective group(s)

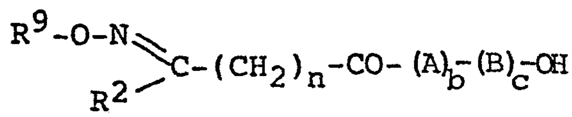 (Ie)

wherein $R^2$, $R^4$, $A^1$, $B^1$, A, B, b, c and n are as defined above and $R^9$ represents an alkyl group.

The substituted oxyiminoalkane acid derivative (Ie) is produced by the reaction of ketocarboxylic acid derivative (XII) and substituted hydroxylamine (XIII) and then releasing the protective group(s).

The reaction proceeds without solvent but is usually performed in an organic (e.g.methanol, ethanol isopropanol, benzene, toluene or xylene) under cooling, at room temperature, or under heating. The releasing of the protective group(s) in the 2nd step may be as in Process A and the reaction conditions are selected according to the starting material, in particular the ester residue.

The compounds of this invention produced by the various processes as described above may be isolated and purified as they are or as salts. The isolation and purification of these compounds may be performed by ordinary methods such as crystallization, distillation, extraction, various chromatographies, recrystallization

The compounds of general formula (I) and the salts thereof provided by the present invention have the structural feature that the fatty acid moiety thereof 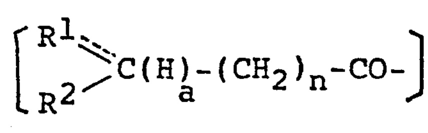 has a carbamide

group, a carbamoyl group, an ether-type or polyether-type group (preferably the group $R^{10}-(O-Y^1)_p-O-Y^2-D-$), or an alkoxyimino group. They have marked fibrinolytic action and can be useful for the prophylaxis and treatment of various thrombotic diseases, such as peripheral arteriovenous occlusion, pulmonary embolism, coronary occlusion, cerebral infarction, myocardial infarction, retinal arteriovenous occlusion, and cancer.

Compounds of this invention, unlike known acylpeptides, have good water solubility, whereby satisfactory pharmacological effect can be expected and the preparation of formulations (e.g. for injection) is facilitated. For example, the solubility of the compound of Example 21 is more than 5mg/ml saline.

The pharmacological actions or effects of compounds of this invention were confirmed by the following methods :

Fibrinolytic Action (Fibrin-clot lysis assay)

Experimental Procedure:

Lots of fibrinogen (1mg/50µl, made by Green Cross Corporation) and respective various amounts of test compounds were added to respective test tubes, and made up in each case to a total volume of 850 µl with cold Tris-buffered saline (20mM)

Tris-buffer, pH 7.4, containing 140 mM NaCl). To the solutions were added 100 µl of Urokinase solution (30 CTA unit/0.1 ml) and 50 µl of thrombin solution (2 NIH units/50 µl) to make a final clot volume of 1 ml each in test tubes in an ice bath. The tubes were quickly shaken to mix the reagents well and immediately placed in a 37°C water bath, and the clot lysis time was recorded. The test compounds were dissolved in physiological saline, except for the comparison which was one dissolved in physiological saline containing 10% cremophore and 0.15% ethanol. The fibrinolytic activity of Urokinase alone was determined and used as a control. By measuring the time of clot lysis, the fibrinolytic activity of each test compound relative to that of Urokinase was determined. The results for formula (I) compounds from subsequent Examples are shown in Table 1 below together with that for a Comparison, 3-hexadecanoylocta-decanoyl-ß-alanyl-L-phenylalanine having the formula

$$\left( \begin{matrix} C_{15}H_{31}COO \\ C_{15}H_{31} \end{matrix} \right\rangle CHCH_2CO\text{-}NHCH_2CH_2CO\text{-}NH\overset{CH_2Ph}{\overset{|}{C}}HCOOH \Bigg) ,$$

which is one of the most active known fibrinolytic compounds.

Experimental Results:

Table 1

| Example No. | Fibrinolytic Action* | Solvent |
|---|---|---|
| 14 | 150% at 1 x $10^{-5}$M | Physiological saline solution |
| 19 | 130-140% at 1 x $10^{-5}$M | " |
| 20 | 130-150% at 1 x $10^{-5}$M | " |
| 21 | 150-160% at 3 x $10^{-6}$M | " |
| 22 | 160% at 3 x $10^{-5}$M | " |
| Comparison: | 140-150% at 1 x $10^{-4}$M | Cremophore solution |

(*): Activity (%) relative to that of Urokinase taken as 100%

In addition, the acute toxicity of the compound of Example 21 was measured as follows. A physiological saline solution of 5 mg/ml of the compound was injected into the veins of the tails of mice at a rate of 0.2 ml/min.; the mice were observed for one week but no dead mice were observed. The toxicity (i.v.) is higher than 200 mg/40 ml saline/kg of mouse, which shows that the compound has almost no toxicity.

Preparations containing compounds of the invention as active component can be produced conventionally using standard carriers and exipients.

The medicament can be administered orally e.g. as tablets,pills, capsules, granules, powders or liquids; by intraveneous or intramuscular injection ; or parenterally . as suppositories .

The dose is determined for each patient (e.g. according to the sympton and the age and sex of the patient) but is usually 5-500 mg/kg, preferably 10-100 mg/kg, per day per adult in the case of intravenous injection, and 5-5000 mg/kg, preferably 10-1000 mg/kg per day per adult in the case of oral administration; the daily dose may be taken all at once, or in e.g. 2 to 4 administrations over the day.

The present invention is further illustrated by the following Examples, in which the following symbols have the following meanings:

Bzl: benzyl group,
BOC: t-butoxycarbonyl group,
Bu: butyl group,
t-Bu: t-butyl group,
HOBT: N-hydroxybenzotriazole
HOSU: N-hydroxysuccinimide
DCC: dicyclohexylcarbodiimide
TFA: trifluoroacetic acid
TsOH: p-toluenesulfonic acid
TEA: triethylamine
DMF: dimethylformamide
DMAP: 4-dimethylaminopyridine
Pd-C: palladium-carbon

Example 1

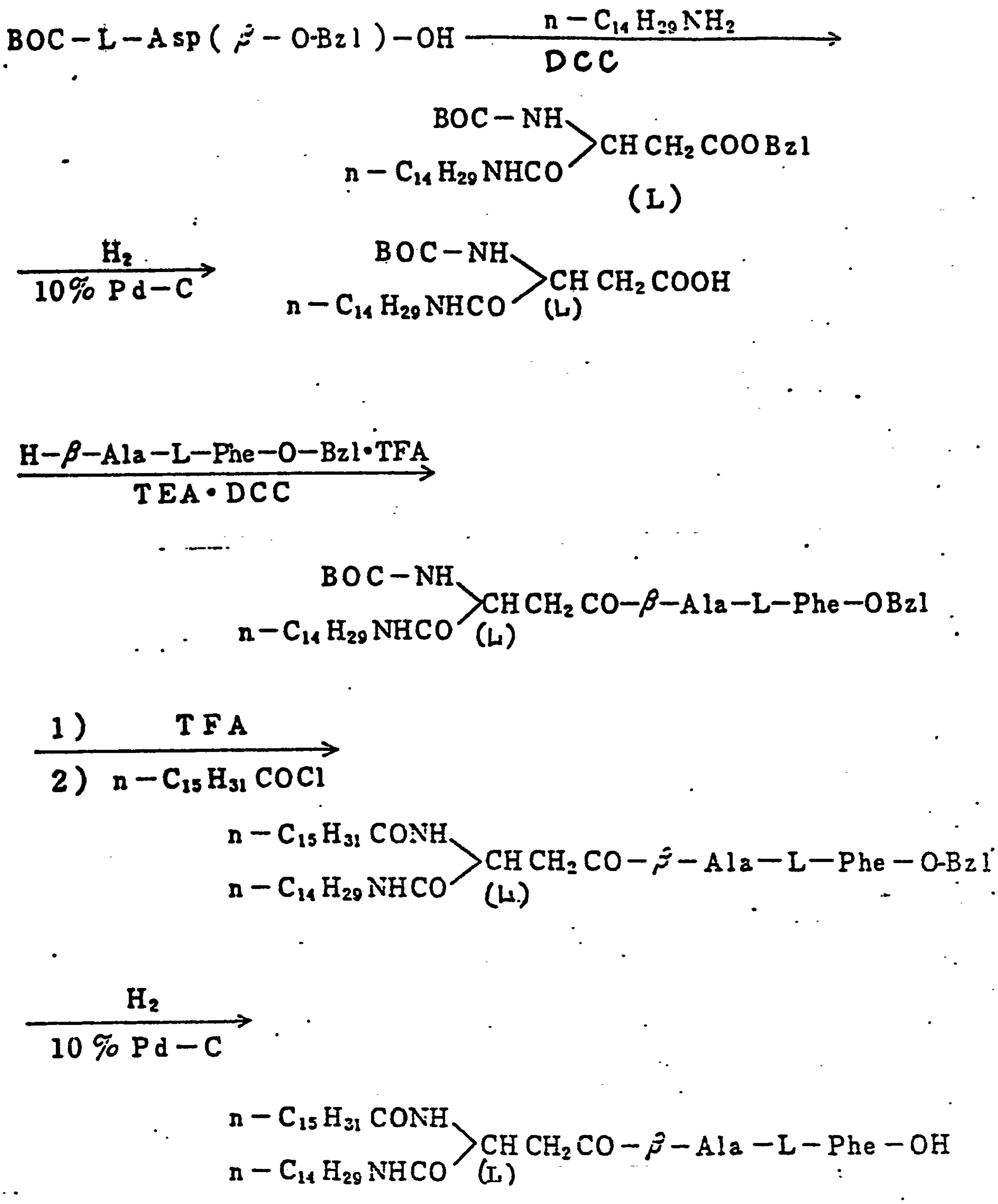
n-C14H29NH2
DCC
H2
10% Pd-C
H-β-Ala-L-Phe-O-Bzl•TFA
TEA•DCC
1) TFA
2) n-C15H31COCl
H2
10 % Pd-C

i) In 50 ml of methylene chloride was dissolved 10 g of t-butoxycarbonyl-L-aspartic acid benzyl ester and after cooling the solution to 0°C and adding thereto 6.6 g of tetradecylamine and 6.4 g of DCC, the mixture was stirred for 2 hours at 0°C and further for 2 days at room temperature. Precipitates thus formed were removed by filtration and the filtrate was washed successively with saline solution, cool dilute HCl, saline solution, 4% aqueous sodium hydrogen carbonate solution and saline solution, and then was dried with anhydrous magnesium sulfate. The solvent in the solution thus obtained was distilled off under reduced pressure and 10.22 g of a white solid material was obtained. The material was dissolved in 130 ml of ethyl acetate and after removing insoluble matters by filtration, the solvent in the filtrate was distilled away under reduced pressure to provide 9.66 g of white powder, L-3-t-butoxycarboamido-3-tetradecylcarbamoylpropionic acid benzyl ester.

IR(KBr) 3310, 2910, 2830, 1730, 1655 $cm^{-1}$

NMR($CDCl_3$, δ) 0.90(3H, t, J=6Hz), 1.1 ~2.0(33H, m), 2.88(2H, dq, J= 11Hz, 7Hz, 6Hz), 3.2(2H, m), 4.5(1H, m), 5.14(2H, s), 5.64(1H, m), 6.44(1H, m), 7.36(5H, s)

ii) 5.2 g of the product obtained as above was dissolved in a mixture of 50 ml of ethyl acetate and 50 ml of methanol and after adding thereto 500 mg of Pd-C, the product was hydrogenated under normal pressure at room temperature. The reaction mixture was filtered and the solvent in the filtrate was distilled off under reduced pressure to provide 4.15 g of white solid L-3-t-butoxycarboamido-3-tetradecylcarbamoyl-propionic acid.

NMR($CDCl_3$, δ) 0.88(3H, t, J = 6Hz), 1.1 ~ 2.0(33H, m), 2.96(2H, m), 3.24(2H, m), 4.48(1H, m), 5.8 (1H, m), 6.7 (1H, m)

iii) In 10 ml of methylenechloride were dissolved 1.59 g of the product obtained at i) above and 1.65 g of β-alanyl-L-phenylalanine benzyl ester TFA salt and after cooling to 0°C and adding 521 μl of TEA and 772 mg of DCC, the mixture was stirred for 1 hour at 0°C, and furter for 24 hours at room temperature.

Precipitate thus formed was removed by filtration, and the filtrate was washed successively with saline solution, cool dilute HCl, saline solution, 4% aqueous sodium hydrogencarbonate solution and saline solution, and was dried over anhyrous magnesium sulfate. The solvent in the solution obtained was distilled away to provide 1.92 g of white solid material N-(L-3-t-butoxycarboamido-3-tetradecyl-carbamoylpropionyl-β-alanyl-L-phenylalanine benzyl ester.

IR(KBr) 3290, 2910, 2830, 1730, 1680, 1635 $cm^{-1}$

NMR($CDCl_3$, δ) 0.86(3H, t, J=6Hz), 1.1~2.0(33H, m), 2.1~2.2(2H, m), 2.6(2H, m), 3.0~3.3(4H, m), 4.4 (1H, m), 4.86(1H, m), 5.2(2H, s), 6.76~7.60(3H, m), 7.24(5H, s), 7.36(5H, s)

iv) In a mixture of 20 ml of methylenechloride and 20 ml of anisole was dissolved 1.9 g of the product obtained at iii) above and after cooling the solution to -10°C and adding dropwise 40 ml of TFA, the mixture was stirred for 2 hours at 0°C. The reaction mixture was concentrated under reduced pressure. Benzene was added to the residue and the formed mixture was concentrated under reduced pressure; after repeating this, the residue was dissolved in 30 ml of methylenechloride and after cooling the mixture to 0°C and adding thereto 824 μl of TEA, and then cooling the mixture to -20°C, a solution of 714 mg of hexadecanoylchloride in 10 ml of methylene chloride was added. The mixture was stirred for 1 hour at 0°C. The reaction mixture was washed successively with saline solution, dilute hydrochloric acid and saline solution and dried over anhydrous magnesium sulfate. The drying agent was removed by filtration, and the filtrate distilled under reduced pressure to remove the solvent.

790 mg of a white powder, N-[(S)-3-tetradecylcarbamoyl-3-hexadecaneamidopropionyl]-β-alanyl-L-phenylalanine benzyl ester, was obtained.

IR(KBr) 3280, 2910, 2830, 1735, 1635 $cm^{-1}$

NMR($CDCl_3$, δ) 0.88(6H, t, J=6Hz), 1.1 ~ 1.8(50H, m), 2.16 ~ 2.8(6H, m), 3.0 ~ 3.34(6H, m), 4.8(2H, m), 5.2 (2H, s), 7.30(5H, s), 7.38(5H, s)

(v) In a mixture of 200 ml of dioxane and 50 ml of methanol was suspended 750 mg of the product obtained at iv) above and after adding thereto 150 mg of 10% Pd-C, the product was hydrogenated under normal pressure at room temperature. The reaction mixture was filtered and the filtrate was concentrated under reduced pressure and the residue formed was subjected to column chromatography (silica gel:100 g; chloroform-methanol from 20:1 to 5:1) to purify the product. 600 mg of a white powder, N-((S)-3-hexadecaneamido-3-tetradecylcarbamoylpropionyl)-β-alanyl-L-phenylalanine, was obtained.

IR(KBr) 3275, 2910, 2830, 1715, 1635, 1540 $cm^{-1}$

mp 190 – 193 ℃

NMR($CDCl_3$ + $CD_3OD$, δ) 0.87(6H, t, J= 6Hz), 1.1 ~ 1.7(50H, m), 2.10 ~ 2.65(6H, m), 3.0 ~ 3.3(6H, m), 4.7(2H, m), 7.26(5H, s)

Example 2

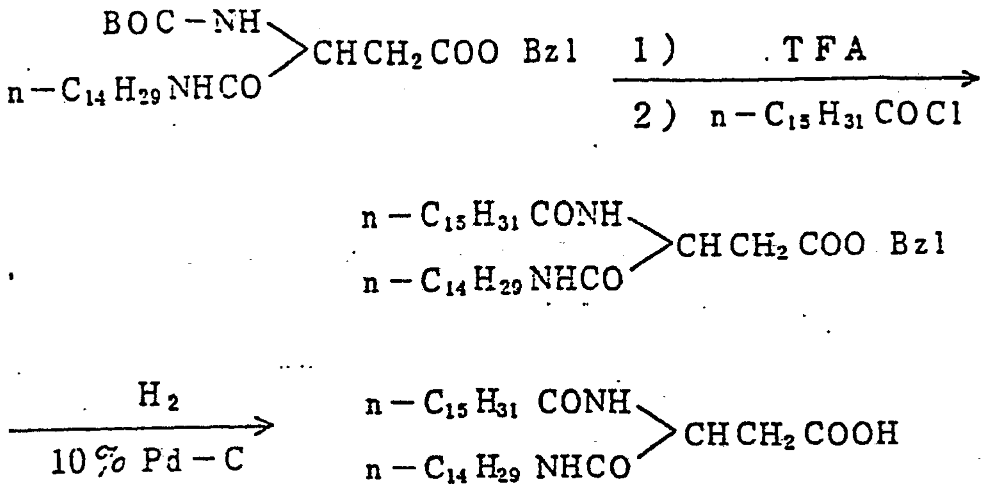


i) By follwing the same procedure as in Example 1-iv) using 1.5 g of the product obtained in the above Example 1-i), 1.86 g of a white powder, L-3-hexadecaneamido-3-tetradecylcarbamoylpropionic acid benzyl ester, was obtained. IR(KBr) 3275, 2910, 2830, 1730, 1635, 1440 $cm^{-1}$

NMR($CDCl_3$, δ) 0.86(6H, t, J=6Hz), 1.1 ~ 1.7(50H, m), 2.2(2H, t, J= 8Hz), 2.82(2H, dq, J=18Hz, 8Hz, 5Hz), 3.18(2H, q, J=6Hz), 4.78(1H, m), 5.16(2H, s), 6.54(1H, m), 6.78(1H, d, J=9Hz), 7.38(5H, s)

ii) By following the same procedure as in Example 1-v) using 1 g of the product obtained in 2 i) above, 800 mg of a white powder, L-3-hexadecaneamido-3-tetradecylcarbamoylpropionic acid, was obtained.

mp. 113 – 115 ℃

IR(KBr) 3275, 2910, 2830, 1720, 1620 $cm^{-1}$

NMR($CDCl_3$ + $CD_3OD$) 0.88(6H, t, J=6Hz), 1.1～1.8(50H, m), 2.22(2H, t, J=9Hz), 2.72(2H, d, J=8Hz), 3.05～3.30(2H, m), 4.72(1H, t, J=8Hz)

## Example 3

$n-C_4H_9(OCH_2CH_2)_3OH$ —— 1) NaH; 2) $BrCH_2COOC(CH_3)_3$ ——→

$n-C_4H_9(OCH_2CH_2)_3OCH_2COOC(CH_3)_3$

—— TFA ——→ $n-C_4H_9(OCH_2CH_2)_3OCH_2COOH$

$BOC-NH$ / $n-C_{14}H_{29}NHCO$ >$CHCH_2COO$-Bzl (L)

—— 1) TFA; 2) $n-C_4H_9(OCH_2CH_2)_3OCH_2COOH$, DCC ——→

$n-C_4H_9(OCH_2CH_2)_3-OCH_2CONH$ / $n-C_{14}H_{29}NHCO$ >$CHCH_2COO$-Bzl (L)

—— $H_2$; 10% Pd–C ——→

$n-C_4H_9(OCH_2CH_2)_3O-CH_2CONH$ / $n-C_{14}H_{29}NHCO$ >$CHCH_2COOH$ (L)

i) In 50 ml of DMF was dissolved 16.48 g of triethyleneglycol monobutyl ether and after cooling the solution to 0°C and adding 3.3 g of sodium hydride (60 % oil solution), the reaction mixture was stirred for 30 minutes at room temperature. The reaction mixture was added dropwise to another solution obtained by dissolving 15.6 g of bromoacetic acid t-butyl ester in 50 ml of DMF and cooling the solution to -10°C. After stirring the mixture for 1 hour at -5°C, for 1 hour at 0°C and for 30 minutes at room temperature, the reaction mixture was distilled under reduced pressure to remove the solvent. Water and ethyl acetate were added to the formed residue to separate the product, and the ethyl acetate layer separated was washed with water, dried over anhydrous magnesium sulfate, and concentrated under reduce pressure to provide 21 g of an oily product. The product was subjected to column chromatography (silica gel:200 g; hexane-ethyl acetate 4:1) to purify the product. 8.6 g of colorless oily of 3,6,9,12-tetraoxahexadecanoic acid t-butyl ester was obtained.

NMR($CDCl_3$, $\delta$) 0.85(3H, t, J=6Hz), 1.1 ~ 1.7(13H, m), 3.43(2H, t, J=6Hz), 3.5 ~ 3.75(12H, m), 3.96 (2H, s)

ii) To the product obtained at i) above was added dropwise 85 ml of TFA while cooling to 0°C and after stirring the mixture for 1 hour at room temperature, the reaction mixture was concentrated under reduced pressure. The residue thus formed was subjected to column chromatography (silica gel:200 g; chloroform-methanol 20:1) to purify the product. 6.4 g of colorless oily of 3,6,9,12-tetraoxahexadecanoic acid was obtained.

NMR($CDCl_3$, δ) 0.8~1.1(3H,m), 1.2~1.7 (4H,m), 3.45(2H,t,J=6Hz), 3.65(12H,t,J=3Hz), 4.13(2H,s)

iii) In a mixture of 20 ml of methylenechloride and 20 ml of anisole was dissolved 3 g of the product obtained at 1-i) above and after cooling the solution to -20°C and adding dropwise thereto 60 ml of TFA, the mixture was stirred for 1 hour at 0°C. The reaction mixture was concentrated under reduced pressure. To the residue was added benzene and the mixture was concentrated under reduced pressure, this being repeated. The product was dried by high vacuum pump. The residue formed was dissolved in 20 ml of methylenechloride and after cooling the solution to 5-10°C and adding thereto a solution of 1.53 g of the product obtained at 3-ii) above in 10 ml of methylenechloride , cooling the mixture to 0°C and adding 0.9 ml of TEA and 1.32 g of DCC, the

reaction mixture was stirred for 3 days at room temperature. Precipitate thus formed was removed by filtration and the filtrate was washed successively with cool dilute HCl, saline solution, 4 % aqueous sodium hydrogencarbonate solution and saline solution, dried over anhyrous magnesium sulfate, and concentrated under reduced pressure to provide 3.6 g of an oily material. The oily material was subjected to column chromatography (silica gel:150 g; chloroform:methanol from 100:1 to 50:1) to purify the product. 2.17 g of colorless oily L-3-tetradecylcarbamoyl-3-(3,6,9,12-tetraoxahexadecaneamido)propionic acid benzyl ester was obtained.

IR(neat) 3290, 2910, 2840, 1730, 1655 $cm^{-1}$

NMR($CDCl_3$, δ) 0.8～1.0(6H,m), 1.2～1.7 (28H,m), 2.84～2.96(2H,m), 3.2(2H,m), 3.45(2H,t,J=6Hz), 3.6～3.8(12H,m), 4.04(2H,s), 4.86(1H,m), 5.16(2H,s), 6.58 (1H,m), 7.36(5H,s), 7.78(2H, d,J=9Hz)

iv) By following the same procedure as in Example 1-ii) using 2.1 g of the product obtained at 3-iii) above, 1.8 g of colorless waxy solid L-3-tetradecylcarbamoyl-3-(3,6,9,12-tetraoxahexadecaneamido)propionic acid was obtained.

IR(KBr) 3280, 2910, 2830, 1725, 1650 $cm^{-1}$

NMR($CDCl_3$, δ) 0.8～1.0(6H, m), 1.16～1.70(28H, m), 2.88(2H, t, J=7Hz), 3.2(2H, m), 3.49(2H, t, J=7Hz), 3.6～3.8(12H, m), 4.06(2H, s), 4.82(1H, m), 6.6～7.0(2H, m), 7.89 (1H, d, J=9Hz)

Example 4

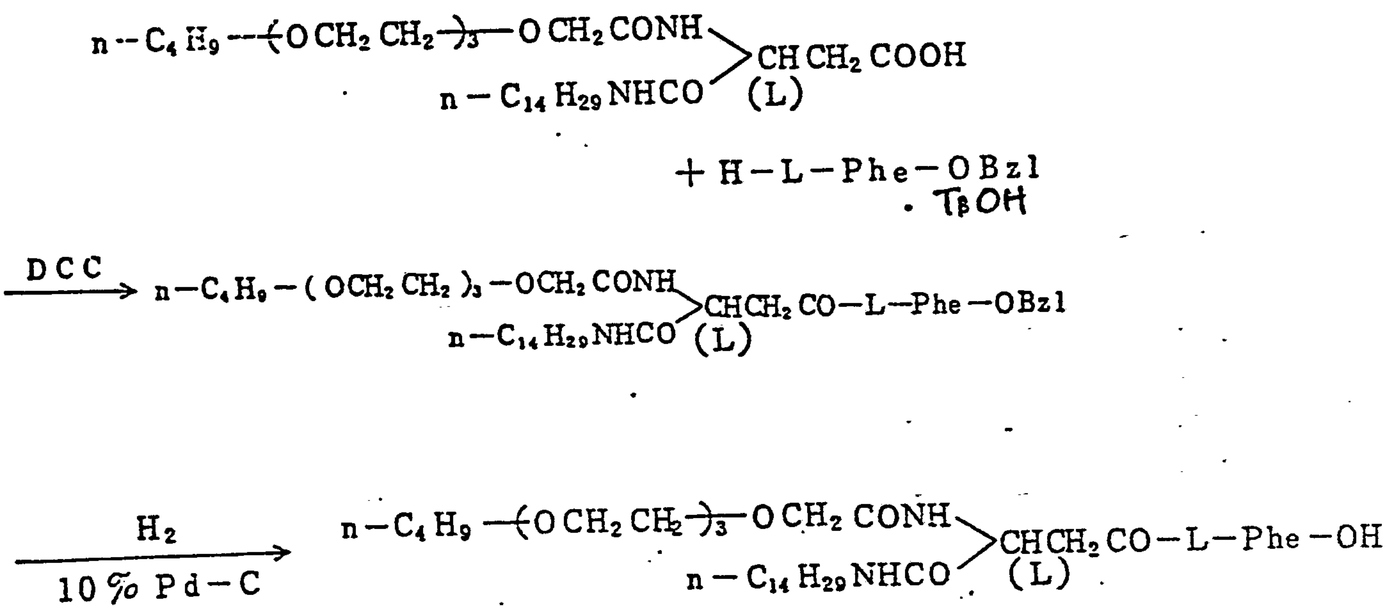

ii) By following the same procedure as in Example 1-iii) using the 1.16 g of the product obtained in Example 3 and 881 mg of L-phenylalanine benzyl ester TsOH salt, 1.4 g of viscous N-[L-3-tetradecylcarbamoyl-3-(3,6,9,12-tetraoxahexadecanamido)propinyl]-L-phenylalanine benzyl ester was obtained.

IR(neat) 3270, 2910, 2840, 1725, 1635 $cm^{-1}$

NMR($CDCl_3$, δ) 0.8～1.0(6H,m), 1.16～1.70 (28H,m), 2.72(2H,m), 3.04～3.25(4H,m), 3.45(2H,t,J=7Hz), 3.55～3.8(12H,m), 4.03(2H,s), 4.6～5.0(2H,m), 5.12(2H,s), 6.57 (1H,d,J=9Hz), 6.86(1H,m), 7.0～7.46(10H,m), 7.98(1H,d,J=9Hz)

ii) By following the same procedure as in Example 1-ii) using the 1.4 g of the product obtained at 4-i) above, 1 g of waxy solid material of N-[3-tetradecylcarbamoyl-3-(3,6,9,12-tetraoxahexadecanamido)propionyl]-L-phenylalanine was obtained.

IR(KBr) 3275, 2910, 2830, 1720, 1630 $cm^{-1}$

NMR($CDCl_3$, δ) 0.8~1.0(6H,m), 1.1~1.7(28H,m), 2.70(2H,t,J=7Hz), 3.0~3.3(4H,m), 3.46(2H,t,J=7Hz), 3.5~3.96(12H,m), 4.02(2H,s), 4.70(2H,m), 6.65(1H,m), 7.04(1H,m), 7.22(5H,s), 7.99(1H,d,J=9Hz)

Example 5

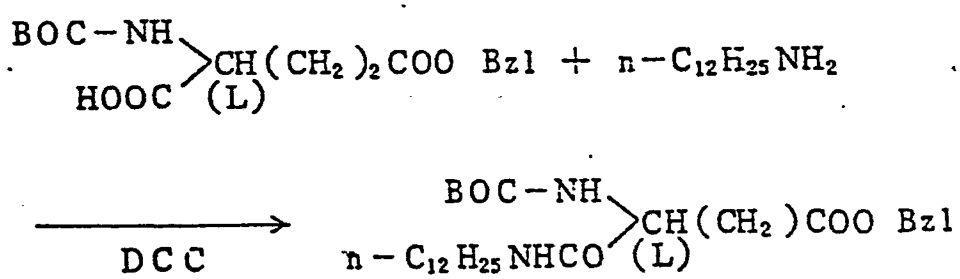

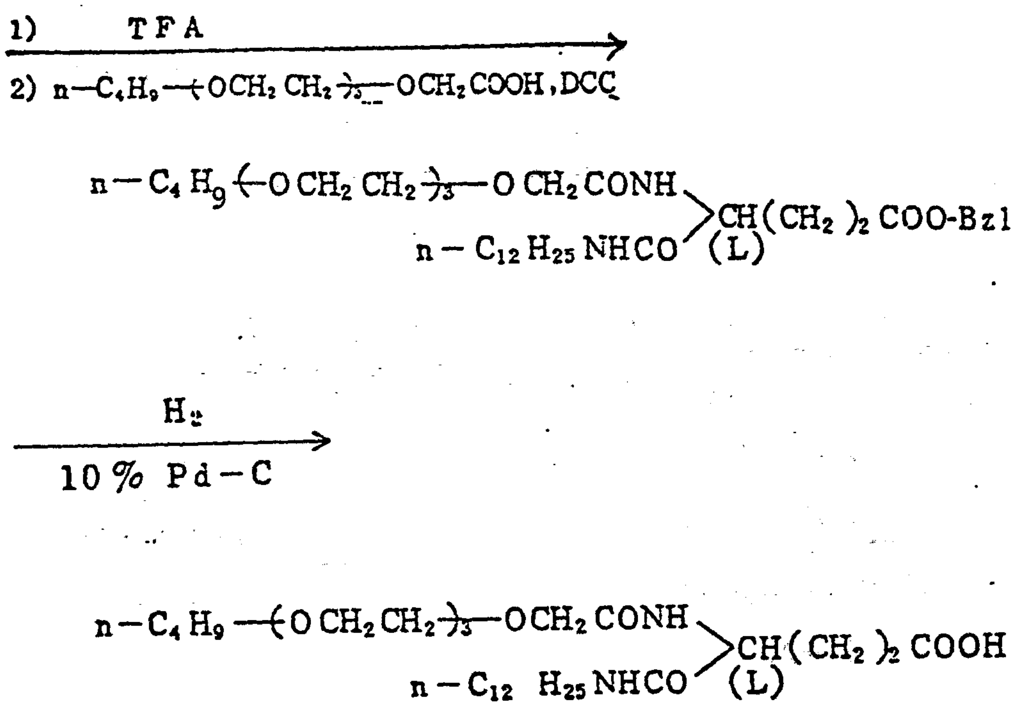


i) By following the same procedure as in Example 1-i) using 10 g of N-t-butoxycarbonyl-L-glutamic acid benzyl ester and 5.5 g of n-decylamine, 2.4 g of a powder, L-4-t-butoxycarboamido-4-dodecylcarbamoyl butyric acid benzyl ester, was obtained.

IR(KBr) 3300, 2900, 2830, 1725, 1675, 1645 $cm^{-1}$

NMR($CDCl_3$, $\delta$) 0.84(3H, t, J=6Hz), 1.1 ~ 1.6(29H, m), 2.0(2H, m), 2.5 (2H, m), 3.23(2H, q, J=7Hz),

4.12(1H,m), 5.15(2H,m), 5.27 (1H, d, J=9Hz), 6.2(1H,m), 7.37(5H,m)

ii) By following the same procedure as in Example 3-iii) using 2.42 g of the product obtained at 5-i) above and 1.27 g of 3,6,9,12-tetraoxahexanoic acid, 1.5 g of colorless oily L-4-dadecylcarbamoyl-4-(3,6,9,12-tetraoxahexanamido)butyric acid benzyl ester was obtained.

IR(KBr) 3275, 2910, 2830, 1730, 1650 $cm^{-1}$

NMR($CDCl_3$, δ) 0.8～1.0(6H,m), 1.15～1.70(24H,m), 2.0～2.25(2H,m), 2.4～2.6(2H,m), 3.22(2H, q, J=7Hz), 3.46(2H, t, J=7Hz), 3.55～3.74(12H,m), 4.02(2H, s), 4.47(1H,m), 5.14(2H, s), 6.34 (1H,m), 7.36(5H, s), 7.47(1H, d, J=10Hz)

iii) By following the same procedure as in Example 1-ii) using 1.4 g of the product obtained at 5-i) above 1.1 g of a white powder, L-4-dodecylcarbamoyl-4-(3,6,9,12-tetraoxahexanamido)butyric acid, was obtained.

IR(KBr) 3350 (broad), 2920, 2840, 1720, 1650 $cm^{-1}$

NMR($CDCl_3$, δ) 0.8 ~ 1.0 (6H, m), 1.06 ~ 1.70 (24H, m), 1.92 ~ 2.12 (2H, m), 2.18 ~ 2.56 (2H, m), 3.22 (2H, q, J=7Hz), 3.47 (2H, t, J=7Hz), 3.55 ~ 3.75 (12H, m), 4.03 (2H, s), 4.64 (1H, m), 7.03 (1H, t, J=7Hz), 7.62 (1H, d, J=10Hz)

Example 6

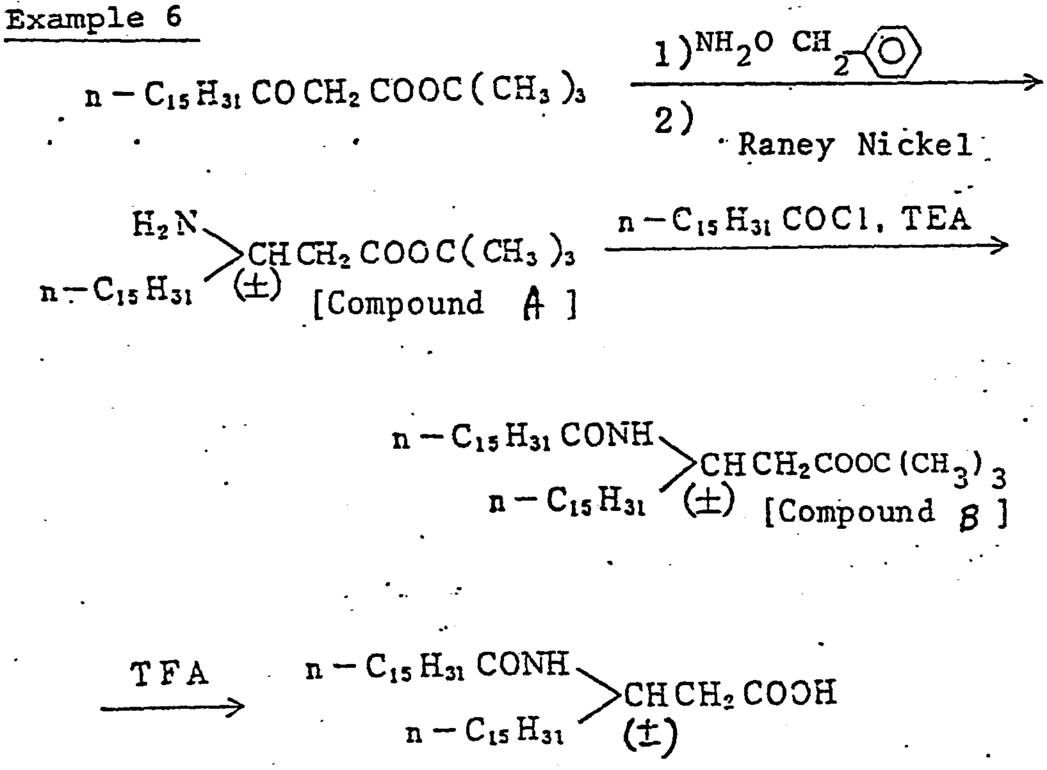


i) In 300 ml of methanol was dissolved 10 g of β-ketooctadecanoic acid t-butyl ester and after adding thereto 4.6 g of O-benzylhydroxylamine hydrochloride and 3.9 ml of TEA, the mixture was refluxed for 1 hour. The mixture was distilled to remove methanol. The residue was dissolved in methylenechloride. The methylenechloride solution thus obtained was washed with cool dilute

hydrochloric acid solution, dried over anyhdrous magnesium sulfate, and distilled under reduced pressure to remove the solvent. 12.8 g of a pale yellow oily material was obtained. The material was dissolved in 100 ml of methanol. To the solution was added a suspension which was obtained by suspending Raney nickel (NIKKO RICA CORPORATION, R-200) in methanol after washing 20 ml of the Raney nickel with water. The product was hydrogenated and after filtering the reaction mixture, the filtrate was distilled under reduced pressure to remove the solvent. The residue was dissolved in methylenechloride The solution thus formed was washed with water, dried over

anhydrous magnesium sulfate, and concentrated under reduced pressure to provide 10 g of a yellow oily material. The product was subjected to column chromatography (silica gel:150 g; chloroform:methanol 20:1) to provide 7.4 g of colorless oily 3-aminooctadecanoic acid t-butyl ester (Compound A). The Compound A was dissolved in 30 ml of methylenechloride and after cooling the solution to -10°C and adding 2.87 ml of TEA, a solution of 5.63 g of hexadecanoyl chloride in 20 ml of methylenechloride was added dropwise thereto. After stirring the mixture for 1 hour, the reaction mixture was poured into ice-water to separate the product. The separated methylene chloride solution was washed successively with saline solution, cool dilute HCl, saline

solution, 5 % aqueous sodium hydrogencarbonate solution, and saline solution, dried over anhydrous magnesium sulfate. The drying agent was removed by filtration, and the filtrate was concentrated under reduced pressure to provide 10.9 g of white solid 3-hexadecanamidooctadecanoic acid t-butyl ester [Compound B].

[Compound A] NMR ($CDCl_3$, δ)

0.87((3H, t, J=6Hz), 1.1~1.4(28H,m), 1.46(9H, s), 2.18(2H,m), 3.1(1H,m),

[Compound B] IR(KBr) 3280, 2910, 2830, 1720, 1635 $cm^{-1}$

NMR ($CDCl_3$, δ) 0.86(6H, t, J=6Hz), 1.1~1.7(63H,m), 2.15(2H, t, J=9Hz), 2.41(2H, d, J=6Hz), 4.2 (1H,m), 6.05(1H, d, J=10Hz)

ii) In 10 ml of methylenechloride was dissolved 10.9 g of Compound B obtained at 6-i) above and after cooling to -10°C and adding dropwise thereto 100 ml of TFA, the mixture was stirred for 1 hour at room temperature. The reaction mixture was concentrated under reduced pressure, and the concentrate was recrystallized from 500 ml of methanol to provide 9.03 g of white crystals of 3-hexadecaneamidooctadecanoic acid.

m.p. 97-98°C

IR(KBr) 3270, 2900, 2830, 1720, 1635, 1550 $cm^{-1}$

NMR($CDCl_3$, $\delta$) 0.88(6H, t, J=6Hz), 1.1 ~ 1.7(54H, m), 2.18(2H, m), 2.48 (2H, d, J=6Hz), 4.2(1H, m)

Example 7

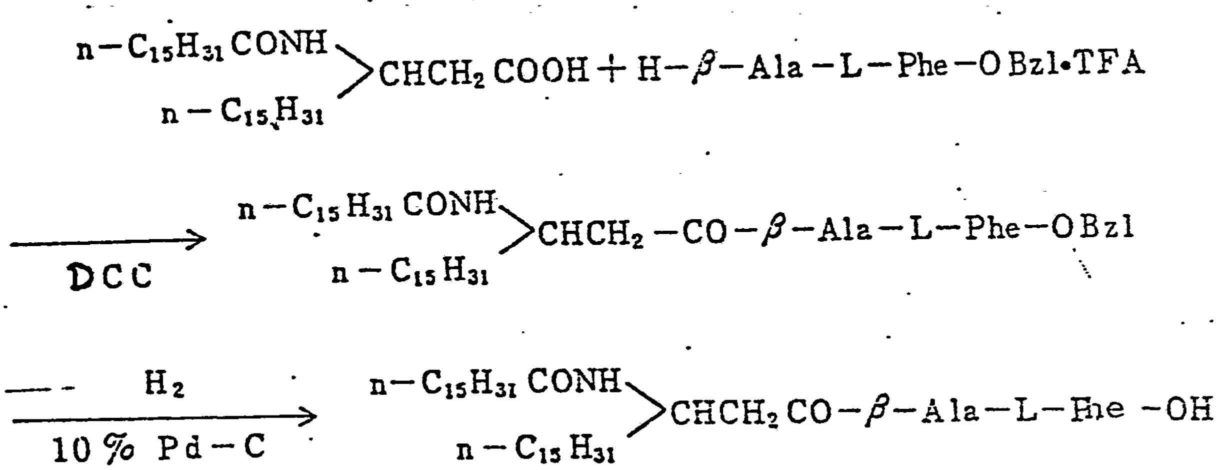


i) In 30 ml of chloroform was suspended 900 mg of 3-hexadecanamidooctadecanoic acid and 810 mg of $\beta$-alanyl-L-phenylalaninebenzyl ester TFA salt and after cooling the suspension to 0°C and adding thereto 256 $\mu$l of TEA and 379 mg of DCC, the mixture was stirred for 30 minutes at 0°C, and then 20 hours at room temperature. To the reaction mixture was added 500 ml of chloroform and after stirring the mixture and removing insoluble matters by filtration, the chloroform solution was washed with successively saline solution, cool dilute HCl, and saline solution, and dried over anhydrous magnesium sulfate. The drying agent was removed by filtration, and the filtrate was distilled under reduced pressure to remove the solvent. 1.85 g of a white solid material was obtained. The material obtained

was subjected to column chromatography (silica gel: 150 g; chloroform:methanol 50:1) to provide 1.07 g of white solid N-(3-hexadecanamidooctadecanoyl)-β-alanyl-L-phenylalanine benzyl ester.

IR(KBr) 3280, 2910, 2840, 1735, 1640, 1440 $cm^{-1}$

NMR($CDCl_3$, δ) 1.90(6H, t, J=6Hz), 1.1 ~ 1.8(54H, m), 2.08 ~ 2.48(6H, m), 3.06 ~ 3.14(4H, m), 4.88(1H, m), 5.2 (2H, s), 7.28(5H, s), 7.38(5H, s)

ii) In 50 ml of methanol and 100 ml of dioxane was suspended 1 g of the compound obtained at 7-i) above and the mixture was hydrogenated over 200 mg of 10 % Pd-C, under normal pressure at room temperature. The reaction mixture was filtered and washed with a mixture of chloform and methanol (1:1). After combining washing solution and the filtrate, the mixture was concentrated under reduced pressure to provide 1 g of grey solid material. The material was dissolved in a mixture of 50 ml of chloroform and 50 ml of methanol, the solution was filtered with Parlite, and the filtrate was concentrated under reduced pressure, and dried to provide white solid of N-(3-hexadecanamidooctadecanoyl)-β-alanyl-L-phenylalanine.

mp. 169 − 174 ℃

IR(KBr) 3275, 2910, 2830, 1715, 1640 $cm^{-1}$

NMR($CDCl_3$ + $CD_3OD$, δ) 0.88(6H, t, J=6Hz), 1.1 ∼ 1.7(54H, m), 2.05 ∼ 2.44 (6H, m), 3.0 ∼ 3.3(4H, m), 4.0 ∼ 4.3(1H, m), 4.7(1H, m) 7.25(5H, m)

Example 8

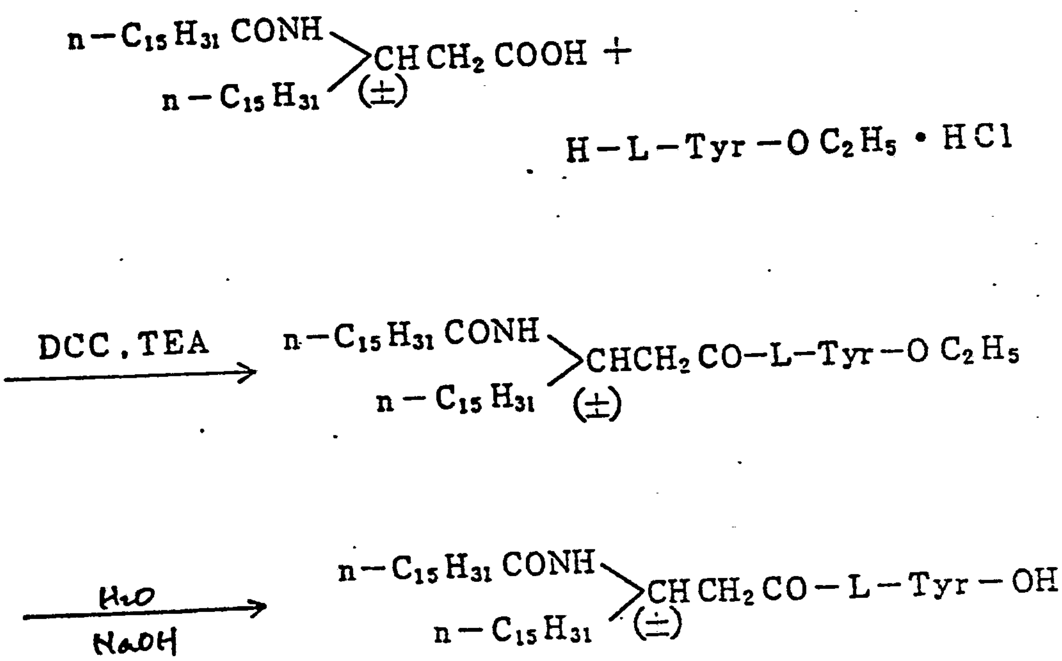

i) By following the same procedure as in Example 7-i) using 1 g of 3-hexadecanamidooctadecanoic acid and 467 mg of L-tyrosine ethyl ester hydrochloride, 1.08 g of white solid N-(3-hexadecanamidooctadecanoyl)-L-tyrosine ethyl ester was obtained.

IR(KBr) 3420, 3270, 2900, 2830, 1715, 1640, 1610 $cm^{-1}$

NMR($CDCl_3$ + $CD_3OD$, δ) 0.88(6H, t, J=6Hz), 1.1~1.7(57H, m), 2.14 (2H, t, J=8Hz), 2.35(2H, d, J=7Hz), 2.98(2H, m), 4.05(1H, m), 4.16(2H, q, J=8Hz), 4.68(1H, t, J=8Hz), 6.64(2H, d, J=10Hz), 7.0(2H, dd, J=10Hz, 2Hz)

ii) In 50 ml of ethanol was dissolved 1.04 g of the compound obtained as above while heating, and after adding thereto 3 ml of 1N sodium hydroxide aqueous solution, the mixture was refluxed for 3 hours. The reaction mixture was concentrated under reduced pressure and after adding to the concentrate 200 ml of water and 600 ml of chloroform, the aqueous layer was acidified to pH 4 with dilute HCl and stirred. Precipitate thus formed was collected by filtration to provide 600 mg of white solid N-(3-hexadecanamidooctadecanoyl)-L-tyrosine.

mp. 141 – 143 ℃

IR(KBr) 3275, 2900, 2830, 1720, 1635 $cm^{-1}$

NMR($CDCl_3$ + $CD_3OD$, δ) 0.88(6H, t, J=6Hz), 1.1 ~ 1.7(54H, m), 2.14(2H, t, J=8Hz), 2.35(2H, d, J=7Hz), 3.03(2H, m), 4.08(1H, m), 4.68 (1H, t, J=7Hz), 6.75(2H, d, J=10Hz), 7.05(2H, dd, J=10Hz, 2Hz)

Example 9

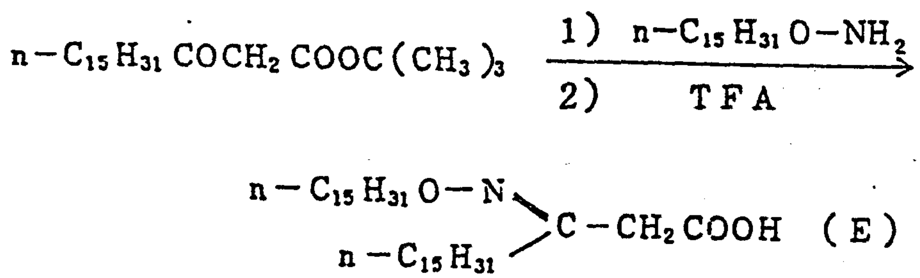


(E)

In 15o ml of methanol were dissolved 4.6 g of 3-oxooctadecanoic acid t-butyl ester and 3.3 g of O-pentadecylhydroxylamine and after refluxing the solution, the mixture was distilled to remove methanol. The residue was dissolved in chloroform and the solution was washed with cool hydrochloric acid and water, dried over anhydrous magnesium sulfate, and concentrated under reduced pressure to provide 7,41 g of colorless oily material. The material was cooled to 0°C and after adding dropwise thereto 80 ml of TFA, the mixture was stirred for 1.5 hour at room temperature. The reaction mixture was concentrated under reduced pressure to provide 6.6 g of white solid material. The material was subjected to column chromatography (silica gel:150 g; first chloroform and then chloroform:melthanol 20:1 v/v to provide 4.8 g of (E)-3-(pentadecyloxyimino)octadecanoic acid (anti isomer).

mp. 65 − 67 ℃

IR(KBr) 2910, 2830, 1685 $cm^{-1}$

NMR($CDCl_3$, δ) 0.87(6H, t, J=6Hz), 1.1 ~ 1.7(52H, m), 2.38(2H, t, J=9Hz), 3.28(2H, s), 4.06(2H, t, 7Hz)

Example 10

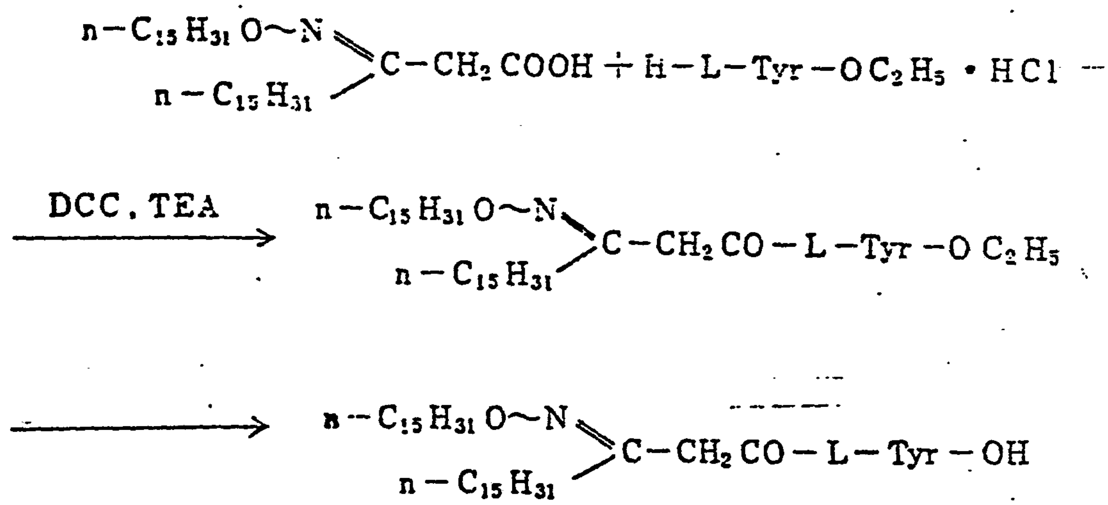


i) In 15 ml of chloroform were dissolved 1 g of 3-pentadecyloxyiminooctadecanoic acid and 480 mg of L-tyrosine ethyl ester hydrochloride and after cooling to 0°C and adding 273 μl of TEA and 402 mg of DCC thereto, the mixture was stirred for 1 hour at 0°C and then 3 days at room temperature. Precipitate thus formed was removed by filtration and the filtrate was washed with successively cool dilute HCl, saline solution, 4 % aqueous hydrogencarbonate and saline solution, dried over anhydrous magnesium sulfate, and concentrated under reduced pressure to provide 1.6 g of a white solid material. The material was recrystallized from a mixture of

methanol and ethyl acetate (1:1) to provide 770 mg of N-[3-(pentadecyloxyimino)octadecanoyl]-L-tyrosine ethyl ester.

IR(KBr) 3400, 3275, 2900, 2830, 1720, 1640 $cm^{-1}$

NMR($CDCl_3$, δ) 0.84(6H, t, J=6Hz), 1.1 ~ 1.7(55H, m), 2.3(2H, m), 3.05 (2H, m), 3.11(2H, s), 4.02(2H, t, J=7Hz), 4.17(2H, q, J=8Hz), 4.82(1H, m), 5.68(1H, s), 6.72 (2H, d, J=10Hz), 6.99(2H, d, J=10Hz), 6.88(1H, m)

ii) In 20 ml of methanol was dissolved the compound obtained at 10-i) above and after adding thereto 2.2 ml of aqueous IN-sodium hydroxide, the mixture was refluxed for 3 hours. The reaction mixture was concentrated under reduced pressure and after adding to the concentrate 100 ml of chloroform and adjusting the pH to 3 with dilute hydrochloric acid, the chloroform layer separated was washed with water, dried over anhydrous magnesium sulfate, and concentrated under reduced pressure to provide 0.8 g of residue. The residue was subjected to column chromatography (silica gel 30 g; chloroform:Methanol from 20:1 to 2:1) to purify the product.

A white waxy material, 470 mg of N-[3-(pentadecyloxyimino)octadecanoyl]-L-tyrosine (a mixture of syn- and anti-isomers), was obtained.

IR(KBr) 3290, 2910, 2830, 1690, 1645 $cm^{-1}$

NMR($CDCl_3$ + $CD_3OD$, δ) 0.88(6H, t, J=6Hz), 1.1~1.7(52H, m), 2.26 (2H, m), 2.98~3.12(4H, m), 4.02(2H, t, J=7Hz), 4.7(1H, t, J=7Hz), 6.75(2H, d, J=10Hz), 7.02(2H, dd, J=10Hz, 3Hz)

Example 11

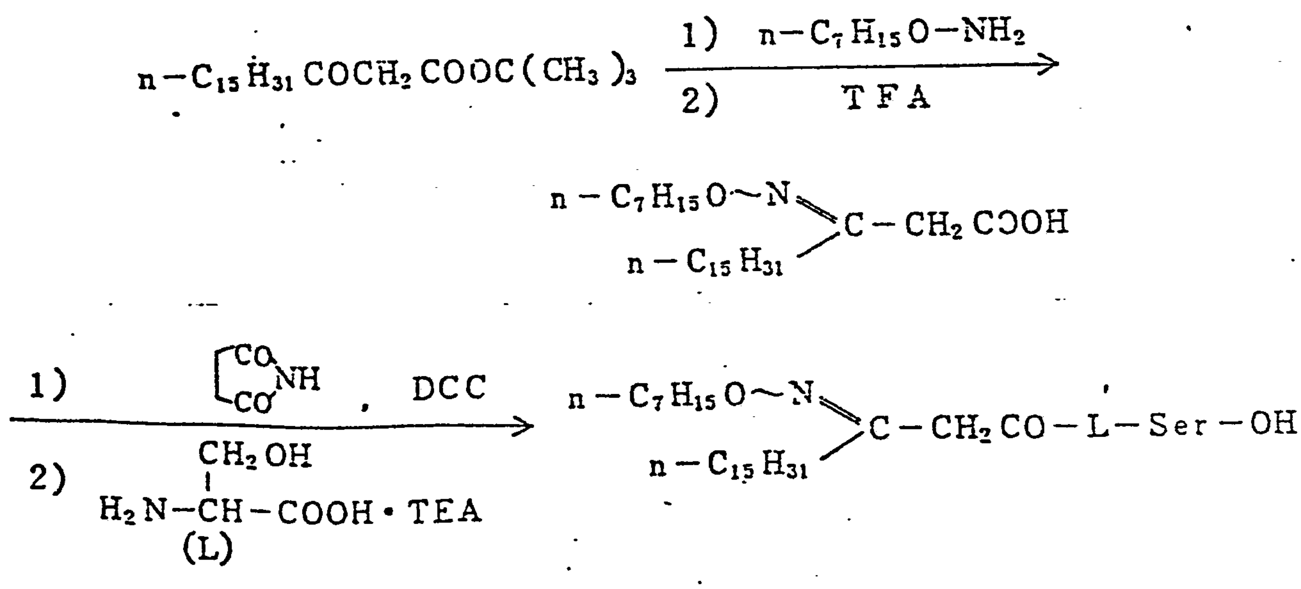


i) By following the same procedure as in Example 9 using 5 g of 3-oxooctadecanoic acid t-butyl ester and O-heptylhydroxylamine, 3 g of a colorless oil, 3-heptyloxyiminooctadecanoic acid (a mixture of syn- and anti-isomers), were obtained.

IR(neat) 2910, 2830, 1705 $cm^{-1}$

NMR($CDCl_3$, δ) 0.8～1.0(6H, m), 1.1～1.7 (36H, m), 2.28(2H, m), 3.23(0.5H, s), 3.3(1.5H, s), 4.02(2H, m)

ii) In 20 ml of dioxane was dissolved 3 g of 3-heptyloxyiminooctadecanoic acid and after adding 0.88 g of HOSU, cooling the mixture to 0°C, and adding 1.57 g of DCC, the mixture was stirred for 1 hour at 0°C and then for 4 hours at room temperature. Precipitate thus formed was removed by filtration, and the filtrate was concentrated under reduced pressure and the residue formed was dissolved in 50 ml of dimethylformamide. The solution was cooled to 0°c and after adding thereto 0.84 g of L-serine and 10 ml of an aqueous solution of 1.12 ml of TEA, the mixture was stirred for 24 hours at room temperature. The reaction mixture was concentrated under reduced pressure, and the residue was dissolved in 50 ml of chloroform, washed with saline solution, cool dilute hydrochloric acid and then saline solution, and dried over anhydrous magnesium sulfate, and concentrated under reduced pressure to provide 3 g of a viscous material. The material was subjected to column chromatography (silica gel:200 g; chloroform:methanol 20:1) to provide 610 mg of white powder, N-[3-(heptyloxyimino)octadecanoyl]-L-serine.

IR(KBr) 3280, 2910, 2830, 1735, 1640 $cm^{-1}$

NMR($CDCl_3$, δ) 0.88(6H, t, J=6Hz), 1.1 ~ 1.7(36H, m), 2.40(2H, m), 3.24 (2H, s), 4.05(2H, t, J=7Hz), 4.1(2H, m), 4.60(1H, m), 7.56 (1H, m)

Example 12

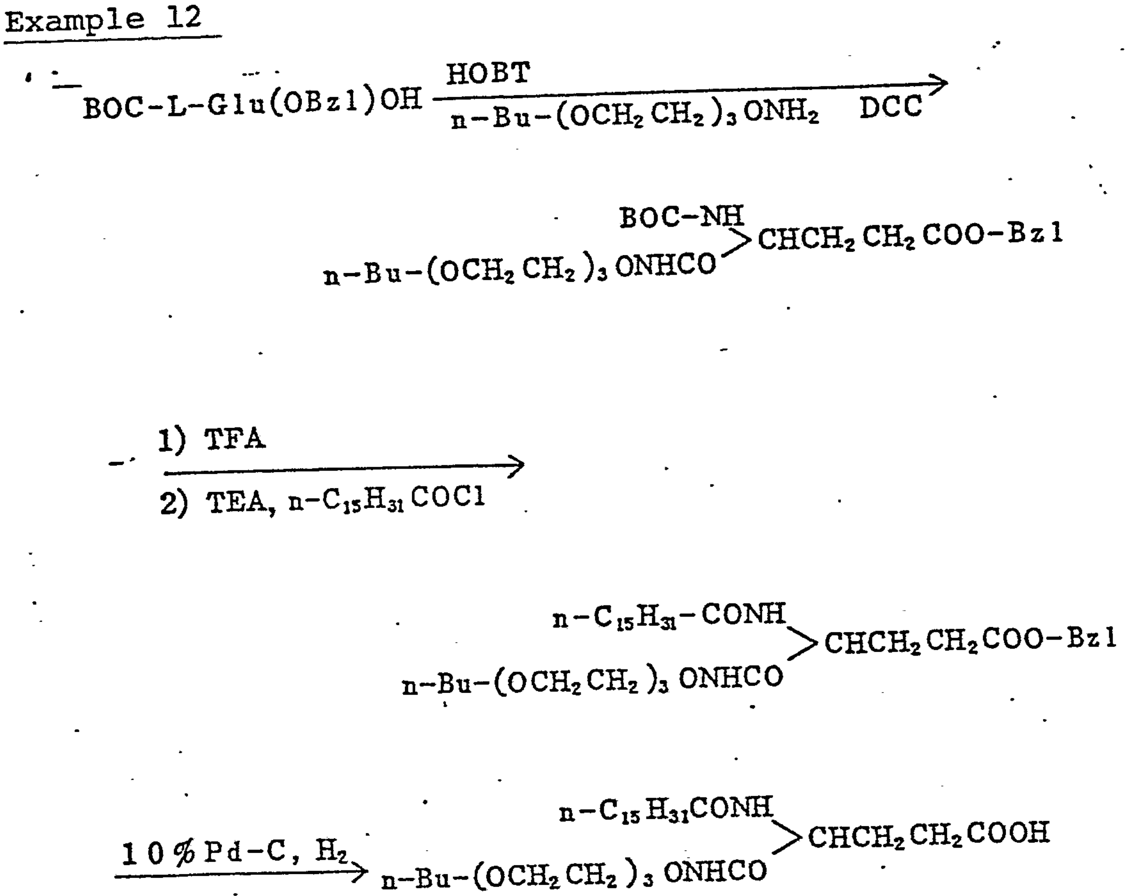


i) In 20 ml of methylenechloride was dissolved 2g of N-t-butoxycarbonyl-L-glutamic acid benzyl ester and 877 mg of HOBT and after cooling to 0°C and adding 1.24 g of DCC, the mixture was stirred for 30 minutes. To the mixture was added 1.33 g of O-(3,6,9-trioxatridecyl)hydroxylamine, and the mixture was stirred

for 1 hour under ice-cooling and then 12 hours at room temperature. Precipitate formed was removed by filtration and the filtrate was distilled under reduced pressure to remove the solvent. The formed residue was subjected to column chromatography (silica gel: 200 g; chloroform:methanol 100:1) to provide 3.18 g of a colorless oil, (S)-γ-t-butoxycarbonylamino-γ-[N-(3,6,9-trioxatridecyloxy)carbamoyl]butyric acid benzyl ester.

IR(neat) 3250, 2950, 2920, 2860, 1680～1730(broad) $cm^{-1}$

NMR($CDCl_3$, δ) 0.90(3H, t, J=7Hz) 1.2～1.7(13H, m) 1.9～2.3(2H, m) 1.4～1.6 (2H, m) 3.47(2H, t, J=7Hz) 3.6～3.8 (10H, m) 4.08(3H, m) 5.13(2H, s) 5.30(1H, d, J=10Hz) 7.37(5H, s)

ii) In 10 ml of methylenechloride was dissolved 1.5 g of the compound obtained at 12-i) above and after adding 10 ml of anisole thereto, the mixture was cooled to -10°c. To the mixture was added dropwise 30 ml of TFA, and the mixture was stirred for 1 hour under ice-cooling. The reaction mixture was concentrated under reduced pressure, and benzene was added to the residure. The solution thus formed was concentrated under reduced pressure. The formed residue was dissolved in 20 ml of methylenechloride and after adding 1.18 ml of TEA under ice-cooling and then cooling the mixture to -30°C, a solution of 770 mg of n-hexadecanoylchloride in 10 ml of methylenechloride was added dropwise to

the mixture. After stirring the mixture for 20 minutes under ice-cooling, the reaction mixture was poured into ice-water. The mixture was acidified with 1N hydrochloric acid, and extracted with 80 ml of chloroform and 40 ml of chloroform successively. The chloroform layers were combined. The chloroform solution was washed twice with saturated saline solution and dried over anhydrous magnesium sulfate. The drying agent was removed by filtration, and the filtrate was distilled under reduced pressure to remove the solvent. The formed residue was subjected to column chromatography (silica gel : 100 g; chloroform-methanol 100:1 v/v) to provide colorless solid 1.5 g of (S)-4-hexadecanamido-4-[N-(3,6,9-trioxatridecyloxy)carbamoyl]-butyric acid benzyl ester.

IR(KBr) 3270, 2910, 2840, 1725, 1635 $cm^{-1}$

NMR ($CDCl_3$, $\delta$) 0.9 (6H, m) 1.2~1.7 (32H, m) 1.9~2.6 (4H, m) 3.47 (2H, t, J=7Hz) 3.6~3.8 (10H, m) 4.04 (2H, m) 4.40 (1H, m) 5.12 (2H, s) 6.44 (1H, d, J=10Hz) 7.36 (5H, s)

iii) To a mixture of 30 ml of ethyl acetate and 30 ml of methanol was dissolved 1.41 g of the compound obtained at 12-ii) above and the solution was hydrogenated over 140 mg of 10% Pd-C, under normal pressure at room temperature. The reaction mixture was filtered, and the filtrate was concentrated under reduce pressure to provide 1.32 g of colorless (S)-4-hexadecanamido-4-[N-(3,6,9-trioxatridecyloxy)carbamoyl]lactic acid.

IR(KBr) 3270, 2910, 2830, 1730, 1700, 1660, 1635 $cm^{-1}$

NMR($CDCl_3$, δ) 0.9 (6H, m) 1.1～1.7 (32H, m) 1.9～2.5 (4H, m) 3.47 (2H, t, J=7Hz) 3.6～3.8 (10H, m) 4.08 (1H, m) 4.54 (1H, m) 6.71 (1H, d, J=10Hz)

Example 13

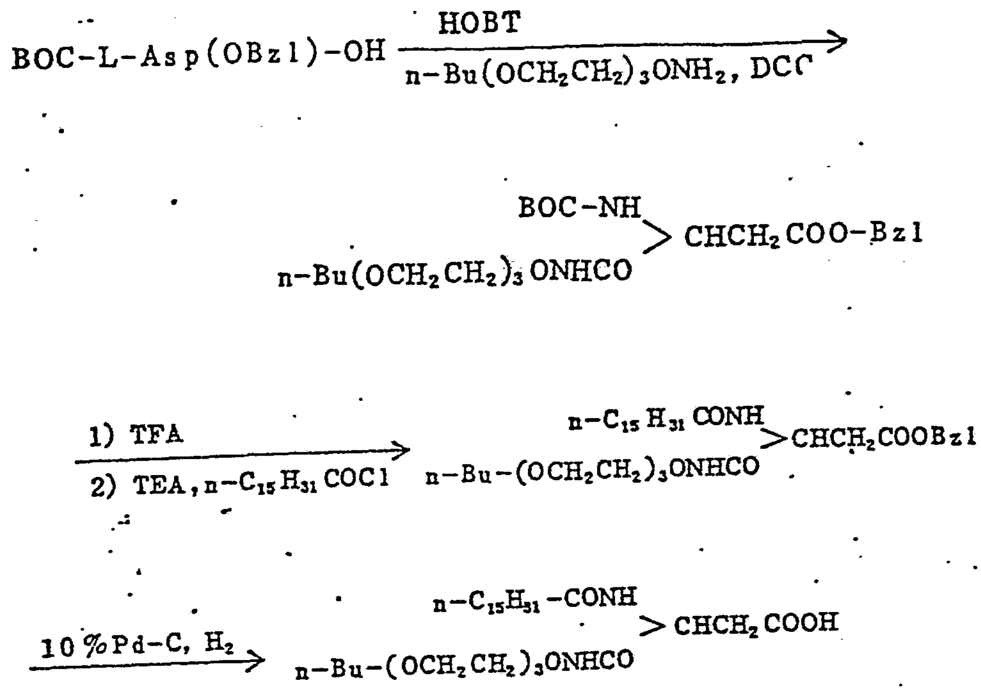


i) By following the same procedure as in Example 1-i) using 4 g of N-t-butoxycarbonyl-L-aspartic acid β-benzyl ester and 2.76 g of O-(3,6,9-trioxatridecyl)-hydroxylamine, 5.88 g of colorless oil, (S)-3-t-butoxycarbonylamino-3-[N-(3,6,9-trioxatridecyloxy)carbamoyl]propionic acid benzyl ester, was obtained.

IR(neat) 3270, 2950, 2920, 2860, 1660～1730(broad) $cm^{-1}$

NMR($CDCl_3$, δ) 0.94(3H, t, J=7Hz) 1.2～1.7(13H, m) 2.87(2H, dd, J=7Hz, 4Hz) 3.48(2H, t, J=7Hz) 3.6～3.8(10H, m) 4.05(2H, m) 4.50(1H, m) 5.15(2H, s) 5.58(1H, d, J=10Hz) 7.37(5H, s)

ii) By following the same procedure as in Example 12-ii) using 1.5 g of the compound obtained at 13-i) above, 1.39 g of colorless solid (S)-3-hexadecanamido-4-[N-(3,6,9-trioxatridecyloxy)carbamoyl]propionic acid benzyl ester was obtained.

IR(KBr) 3270, 3220, 2900, 2830, 1720, 1630 $cm^{-1}$

NMR($CDCl_3$, $\delta$) 0.9(6H, m) 1.2~1.7(30H, m) 2.2(2H, t, J=8Hz) 2.84(2H, m) 3.47 (2H, t, J=7Hz) 3.6~3.8(10H, m) 4.05(2H, m) 4.77(1H, m) 5.15(2H, s) 6.75(1H, d, J=8Hz) 7.37(5H, s)

iii) By following the same procedure as in Example 12-iii) using 1.39 g of the compound obtained at 13-ii) above, 1.0 g colorless solid (S)-3-hexadecanamido-4-[N-3,6,9-trioxatridecyloxy)carbamoyl]propionic acid was obtained.

IR(KBr) 3270, 3200, 2910, 2830, 1700, 1645 $cm^{-1}$

NMR($CDCl_3$) 0.9 (6H, m) 1.2 ~ 1.7 (30H, m) 2.24 (2H, m) 2.80 (2H, m) 3.48 (2H, t, J=7Hz) 3.6 ~ 3.8 (10H, m) 4.07 (2H, m) 4.80 (1H, m) 6.90 (1H, d, J=9Hz)

Example 14

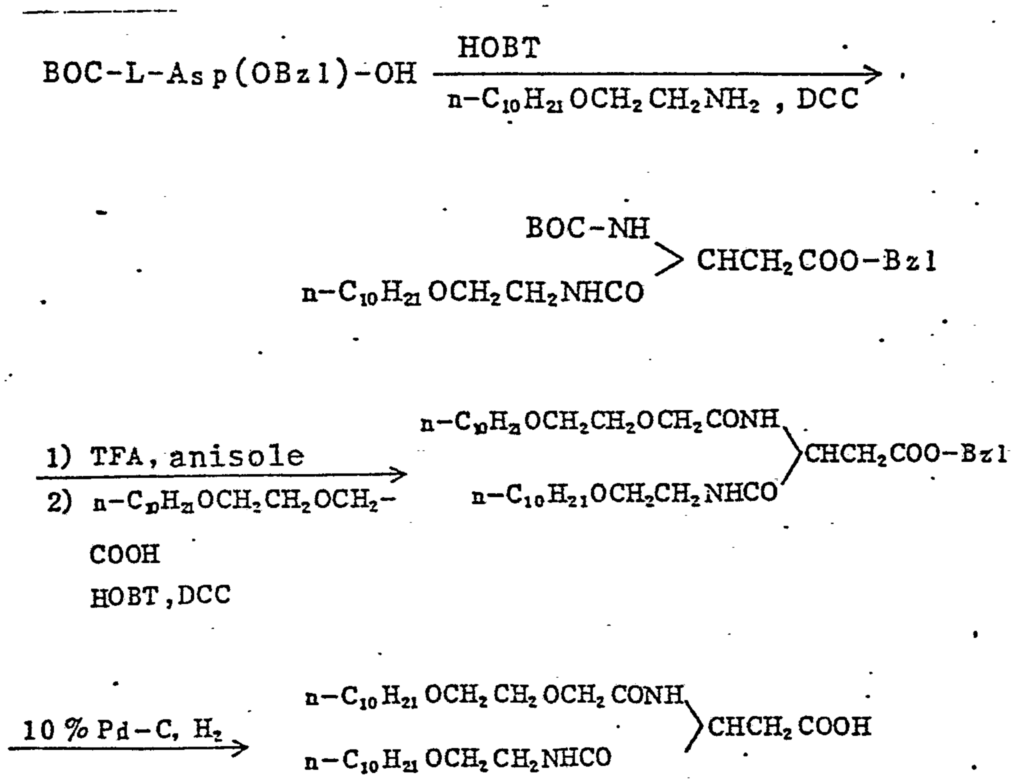

i) By following the same procedure as in Example 12-i) using 3.05 g of N-t-butoxycarbonyl-L-aspartic acid β-benzyl ester and 3.05 g of O-decylethanolamine, 5.58 g of colorless oil, (S)-3-t-butoxycarbonylamino-3-(3-oxatridecylcarbamoyl)propionic acid benzyl ester, was obtained.

IR(neat) 3280, 2920, 2840, 1715, 1665$cm^{-1}$

NMR($CDCl_3$, δ) 0.87(3H, t, 6Hz), 1.2～1.7(25H, m) 2.7(1H, dd, J=7Hz, 19Hz) 3.10(1H, dd, J=5Hz, 19Hz) 3.3～3.5(6H, m) 4.5(1H, m) 5.12(2H, s) 5.62(1H, m) 6.75(1H, m) 7.35 (5H, s)

ii) In 5 ml of methylenechloride was dissolved 800 mg of the compound obtained at 14-i) above, and after adding 5 ml of anisole and adding dropwise 10 ml of TFA under ice-cooling, the mixture was stirred for 1 hour under ice-cooling. The reaction mixture was concentrated under reduce pressure. To the formed residue was added 50 ml of ether and 50 ml of water, and the pH of the mixture was adjusted to 9-10 with 4 % aqueous sodium hydrogencarbonate. The aqueous layer separated was extracted with 30 ml of ether and after combining the ether layers separated, the ether solution was washed with a saturated saline solution, dried over anhydrous magnesium sulfate, and concentrated under

reduced pressure to provide a colorless solid material.

411 mg of 3,6-dioxahexadecanoic acid and 216 mg of HOBT were dissolved in 10 ml of methylenechloride and after adding thereto 330 mg of DCC under ice-cooling , the mixture was stirred for 30 minutes under ice-cooling. To the reaction mixture was added a solution of the above colorless solid material in 10 ml of methylenechloride, and the mixture was stirred for 24 hours at room temperature. The reaction mixture was filtered and the filtrate was washed succesively with 0.1 N hydrochloric acid , a saturated saline solution, aqueous 4% sodium hydrogencarbonate solution and saturated saline solution, dried over anhydrous magnesium sulfate, and concentrated under reduced pressure to provide a colorless solid material. The material was subjected to column choromatography (silica gel:30 g; chloroform:methanol 100 v/v) to provide colorless solid (840 mg). (S)-3-(3,6-dioxahexadecanamido)-3-(3-oxatridecylcarbamoyl)-propionic acid benzyl ester.

IR(KBr) 3290, 2920, 2840, 1730, 1645 $cm^{-1}$

NMR($CDCl_3$, δ) 0.88(6H, m) 1.2～1.7(32H, m) 2.9(2H, m) 3.32～3.70(12H, m) 4.03 (2H, s) 4.90(1H, m) 5.15(2H, s) 6.75 (1H, m) 7.35(5H, s) 7.72(1H, d, J=10Hz)

iii) By following the same procedure as in Example 12-iii) using 830 mg of the compound obtained 14-ii) above, 620 mg of colorless solid (S)-3-(3,6-dioxahexadecanamido)-3-(3-oxatridecylcarbamoyl)propionic acid was obtained.

m.p. 70～71 ℃

IR(KBr) 3320, 3275, 2910, 2840, 1710, 1675 $cm^{-1}$

NMR($CDCl_3$, δ) 0.88(6H, m) 1.2～1.7(32H, m) 2.87(2H, m) 3.35～3.70(12H, m) 4.06 (2H, s) 4.88(1H, m) 6.94(1H, m) 7.76 (1H, d, J=10Hz)

Example 15

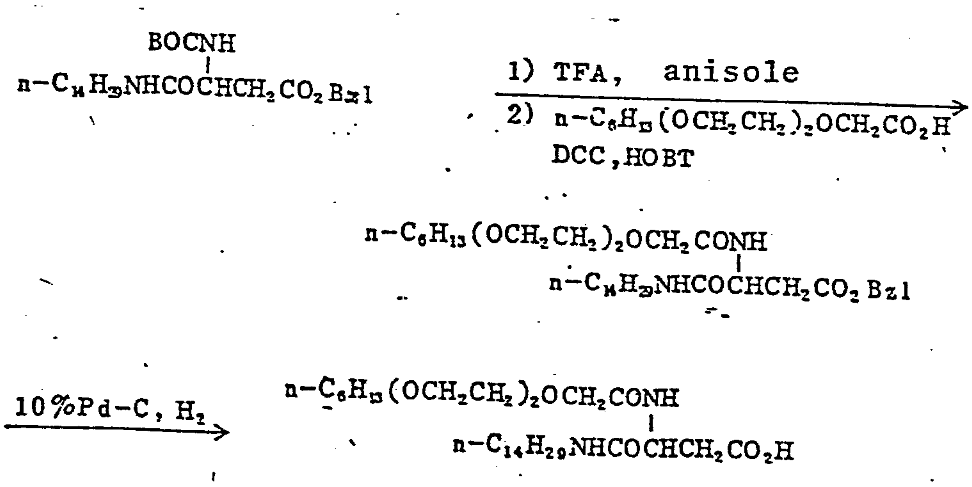

i) In 13 ml of methylenechloride was dissolved 2 g of the compound obtained by Example 1-i) and after adding thereto 13 ml of anisole and adding dropwise thereto 40 ml of TFA under ice-cooling, the mixture was stirred for 2 nours under ice-cooling . The reaction mixture was concentrated under reduced pressure to provide a residue. In 10 ml of methylenechloride were dissolved 957 mg of 3,6,9-trioxahexadecanoic acid and 540 mg of HOBT, and after adding thereto 803 mg of DCC under ice-cooling, the mixture was stirred for 30 mintes. To the mixture were added a solution of the above residue and 560 μl of TEA in 10 ml of methylenechloride, and the mixture was stirred for 24 hours at room temperatuee. The reaction mixture was filtered, and the filtrate was washed successively with 0.5 N-hydrochloric acid, saturated saline solution, 4 % aqueous sodium hydrogencarbonate, and saturated saline solution, and after drying over anhydrous magnesium sulfate the solution was concentrated under reduced pressure to provide an oily material. The material was subjected to column chromatography (silica gel:100 g; ethyl acetate-hexane 1;2 v/v) to provie 1.06 g of colorless solid material of (S)-3-tetradecylcarbamoyl-3-(3,6,9-trioxahexadecanamido)propionic acid benzyl ester.

IR(KBr) 3300, 2920, 2840, 1730, 1660 $cm^{-1}$

NMR($CDCl_3$, δ) 0.88(6H, m) 1.2～1.7(32H, m) 2.90(2H, m) 3.21(2H, m) 3.46(2H, t, J=7Hz) 3.6～3.78(8H, m) 4.04(2H, s) 4.87(1H, m) 5.16(2H, s) 6.54(1H, m)

ii) By following the same procedure as in Eample 12-iii) using 1 g of the compound obtained at 15-i) above, 750 mg of white solid. (S)-3-tetradecylcarbamoyl-3-(3,6,9-trioxahexadecanamido)propionic acid was obtained.

IR(KBr) 3290, 2910, 2830, 1715, 1650, 1630 $cm^{-1}$

NMR($CDCl_3$, δ) 0.88(6H. m) 1.2～1.7(32H, m) 2.76(1H, dd, J=7Hz, 18Hz) 3.03(1H, dd, J=6Hz, 18Hz) 3.24(2H, m) 3.53(2H, t, J=7Hz) 3.6～3.8(8H, m) 4.06(2H, d, J=3Hz) 4.82(1H, m) 6.60(1H, m) 7.87(1H, d, J=10Hz)

Example 16

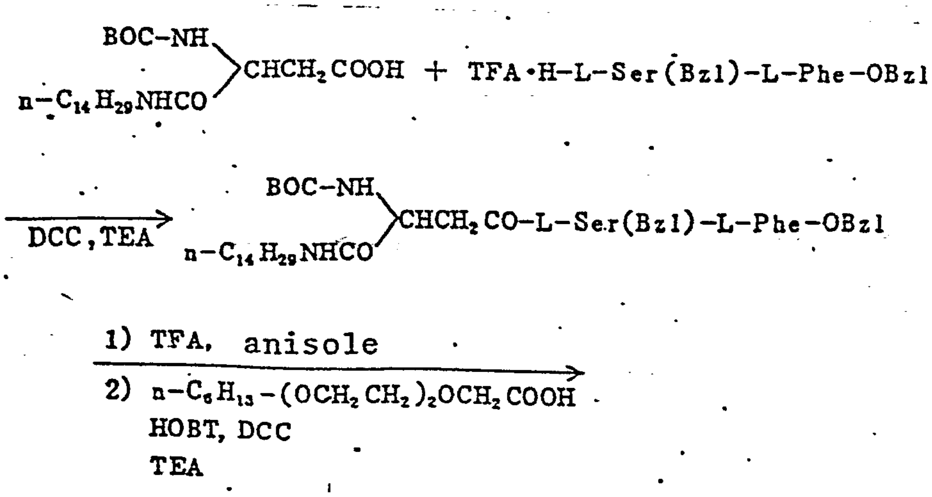

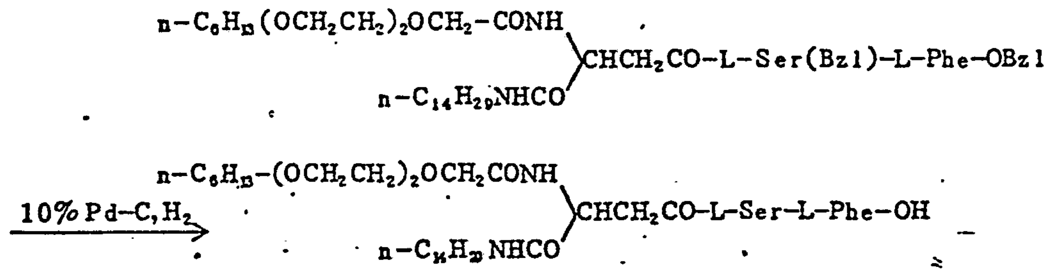


i) In 20 ml of methylenechloride were dissolved 1.73 mg of the compound obtained by Example 1-ii) and 2.21 g of L-seryl-L-phenylalanine benzyl ester TFA salt and after adding thereto 0.57 ml of TEA and 845 mg of DCC under ice-cooling the mixture was stirred for 24 hours at room temperature. To the reaction mixture was added chloroform upto 300 ml, and precipitates were removed by filtration. The filtrate was washed successively with cool 0.5 hydrochloric acid, saturated saline solution, 4% aqueous sodium hydrogencarbonate solution, and saturated saline solution, dried with anhydrous sodium sulfate, and concentrated under reduced pressure. The formed residue was subjected to column chromatography (silica gel:100 g; chloroform-methanol 100:1 v/v) to provide 1.97 g of white solid N-[(S)-3-t-butoxycarboamido-3-tetradecylcarbamoylpropionyl]-L-O-benzylseryl-L-phenylalanine benzyl ester.

IR(KBr) 3280, 2910, 2840, 1720, 1685, 1635 $cm^{-1}$

NMR($CDCl_3$, δ) 0.88(3H, t, J=6Hz) 1.08~1.70(33H, m) 2.50(1H, dd, J=7Hz, 17Hz) 2.85(1H, dd, J=5Hz, 17Hz) 3.0~3.3(4H, m) 3.48(1H, dd, J=10Hz, 8Hz) 3.86(1H, dd, J=10Hz, 5Hz) 4.3~4.6(2H, m) 4.45(2H, s) 4.85(1H, m) 5.12(2H, s) 6.0(1H, m) 6.56(1H, m) 6.76(1H, m) 6.9~7.44(15H, m)

ii) By following the same procedure as in Example 15-i) using 980 mg of the compound obtained at 16-i) above and 293 mg of 3,6,9-hexadecanoic acid, 900 mg of white solid N-[(S)-3-tetradecylcarbamoyl-3-(3,6,9-trioxadecanamido)propionyl]-L-O-benzylseryl-L-phenylalanine benzyl ester was obtained.

IR(KBr) 3270, 2920, 2840, 1715, 1635 $cm^{-1}$

NMR($CDCl_3$, δ) 0.88(6H, m) 1.16~1.70(32H, m) 2.68(2H, m) 3.0~3.24(4H, m) 3.26~3.7(11H, m) 3.87(1H, dd, J=4Hz, 10Hz) 4.03(2H, s) 4.44(2H, s) 4.48(1H, m) 4.68~4.96(2H, m) 5.15(2H, s) 7.0~7.4(15H, m)

iii) In 20 ml of acetic acid was dissolved 900 mg of the compound obtained at 16-ii) above and the solution was hydrogenated over 500 mg of 10 % Pd-C under normal pressure at room temperature. The reaction mixture was filtered, and the filtrate was concentrated under reduced pressure to provide 560 mg of

white solid N-[(S)-3-tetradecylcarbamoyl-3-(3,6,9-trioxahexadecanamido)propionyl]-L-seryl-L-phenylalanine.

m. p. 140－142 ℃

IR(KBr) 3275, 2910, 2840, 1720, 1635 $cm^{-1}$

NMR($CDCl_3$, δ) 0.88(6H, m) 1.1～1.7(32H, m) 2.68(2H, m) 3.16(4H, m) 3.37～3.80(12H, m) 3.96(2H, s) 7.22(5H, s)

Example 17

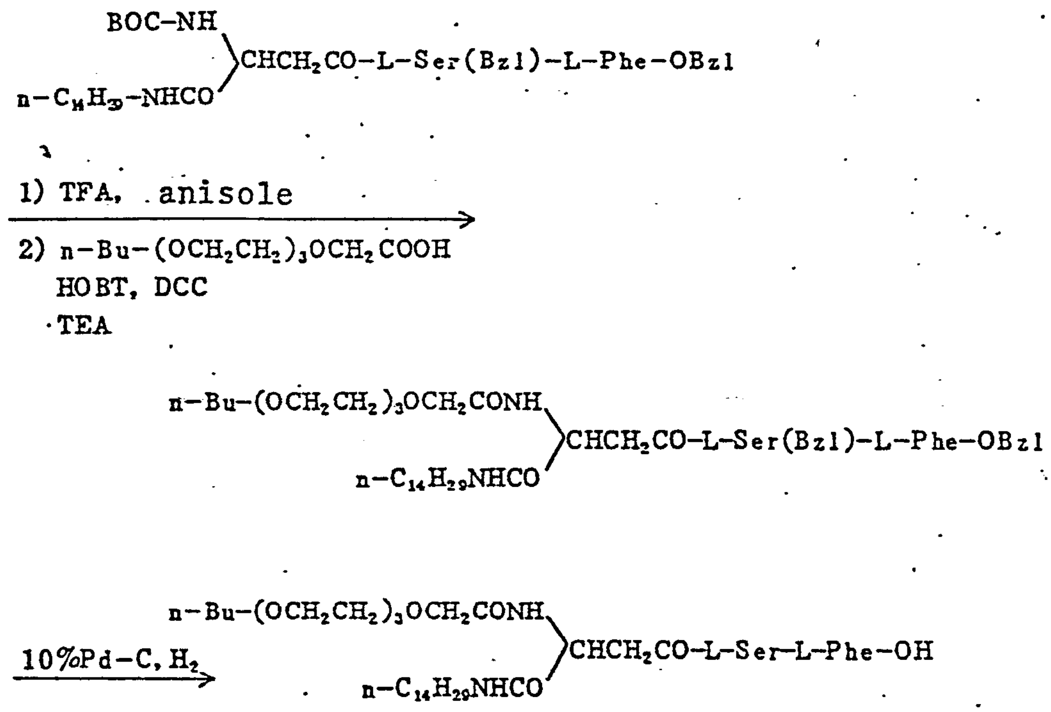


i) By following the same procedure as in Example 15-i) using 970 mg of the compound obtained in Example 16-i) and 304 mg of 3,6,9,12-tetraoxahexadecanoic acid, 1.08 g of white solid N-[(S)-3-tetradecylcarbamoyl-3-(3,6,9,12-tetraoxahexadecanamido)propionyl]-L-O-benzylseryl-L-phenylalanine benzyl ester was obtained.

IR(KBr) 3270, 3060, 2910, 2840, 1715, 1635 $cm^{-1}$

NMR($CDCl_3$, δ) 0.8～1.0(6H, m) 1.15～1.65 (28H, m) 2.7(2H, m) 3.0～3.2(4H, m) 3.44(2H, t, J=7Hz) 3.4～3.7(13H, m) 3.88(1H, dd, J=4Hz, 10Hz) 4.03(2H, s) 4.44(2H, s) 4.5(1H, m) 4.72～5.00(2H, s) 7.0～7.6(15H, m)

ii) By following the same procedure as in Example 16-iii) using 760 mg of the compound obtained at 17-i) above, 500 mg of white solid N-[(S)-3-tetradecylcarbamoyl-3-(3,6,9,12-tetraoxahexadecanamido)-propinyl]-L-seryl-L-phenylalanine was obtained.

IR(KBr) 3270, 3060, 2910, 2840, 1720, 1630 $cm^{-1}$

NMR($CDCl_3$, δ) 0.8～1.0(6H, m) 1.0～1.7 (28H, m) 2.7(2H, m) 3.16(4H, m) 3.3～3.9(16H, m) 3.98(2H, s) 4.4～5.0(3H, m) 7.21(5H, s)

Example 18

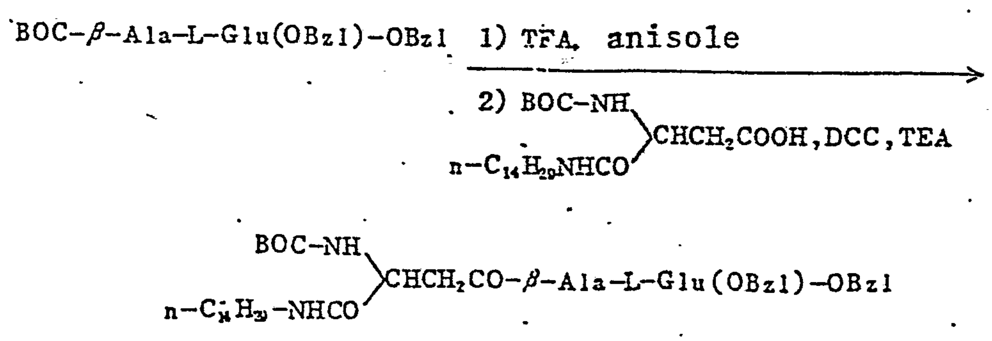

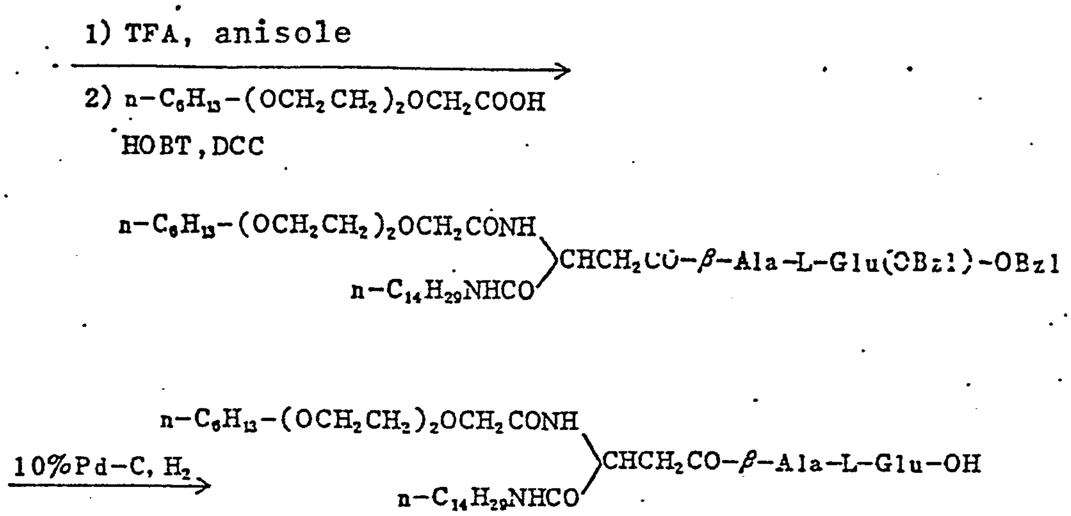


i) In 16 ml of methylenechloride was dissolved 1.63 g of N-t-butoxycarbonyl-β-alanyl-L-glutamic acid α,γ-dibenzylester and after adding thereto 16 ml of anisole, cooling the mixture to -30°C and adding dropwise thereto 32 ml of TFA, the mixture was stirred for 2 hours under ice-cooling. The mixture was concentrated under reduced pressure, and 50 ml of ether and 50 ml of hexane were added to the residue to treat it. The upper layer was removed by decantation, and insoluble β-alanyl-L-glutamic acid dibenzyl ester TFA salt was obtained. In 20 ml of methylenechloride were dissolved the above diester and 1.4 g of the compound obtained by Example 1-ii) and after adding thereto 458 μl of TEA and 680 mg of DCC under cooling, the mixture was stirred for 3 days at room temperature. The reaction mixture was filtered, and the filtrate was washed successively with cool 0.5 N hydrochloric acid, a saturated saline solution, 4 % aqueous sodium hydrogencarbonate, and saturated soline solution, dried over

anhydrous sodium sulfate, and concentrated under reduced pressure to provide a white solid material. The material was subjected to column chromatography (silica gel:100 g; chloroform:methanol 100:1 v/v) to provide 1.7 g of white solid N-[3-t-butoxycarboamido-3-tetradecylcarbamoylpropionyl]-β-alanyl-L-glutamic acid dibenzyl ester.

IR(KBr) 3290, 2910, 2840, 1730, 1685, 1640 $cm^{-1}$

NMR($CDCl_3$, δ) 0.88(3H, t, J=6Hz) 1.15～1.60(24H, m) 1.44(9H, s) 2.10～2.76(8H, m) 2.85～3.30(4H, m) 3.9(1H, m) 4.39(1H, m) 4.67(1H, m) 5.10(2H, s) 5.16(2H, s) 6.88(1H, m) 7.31(5H, s) 7.34(5H, s) 7.74 (1H, d, J=9Hz)

ii) By following the same procedure as in Example 15-i), using 1.7 g of the compoud obtained at 18-i) above and

523 mg of 3,6,9-trioxahexadecanoic acid, 1.36 g of white N-[(S)-3-(3,6,9-trioxahexadecanamido)-3-tetradecylcarbamoylpropionyl]-β-alanyl-L-glutamic acid dibenzyl ester was obtained.

IR(KBr) 3280, 3070, 2910, 2840, 1730, 1635 $cm^{-1}$

NMR($CDCl_3$, δ) 0.88(6H, m) 1.05～1.70(32H, m) 2.0～2.8(8H, m) 1.9～2.3(4H, m) 3.46(2H, t, J=7Hz) 3.63(4H, m) 3.75(4H, s) 4.08(2H, s) 4.5～4.84(3H, m) 5.11(2H, s) 5.19(2H, s) 7.02(1H, m) 7.36(5H, s) 7.38(5H, s) 7.75(1H, d, J=10Hz) 8.90(1H, d, J=9Hz)

iii) By following the same procedure as in Ecample 12-iii) using 1.3 g of the compound obtained at 18-ii) above, 1.0 g of white solid N-[(S)-3-tetradecylcarbamoyl-3-(3,6,9-trioxahexadecanamido) propionyl]-β-alanyl-L-glutamic acid was obtained.

IR(KBr) 3275, 3075, 2910, 2840, 1730, 1635 $cm^{-1}$

NMR($CDCl_3$, δ) 0.88(6H, t, J=7Hz) 1.1～1.7(32H, m) 1.9～2.9(8H, m) 3.2(4H, m) 3.48(2H, t, J=7Hz) 3.5～3.8(8H, m) 4.06(2H, s) 4.62(1H, m) 4.85(1H, m)

Example 19

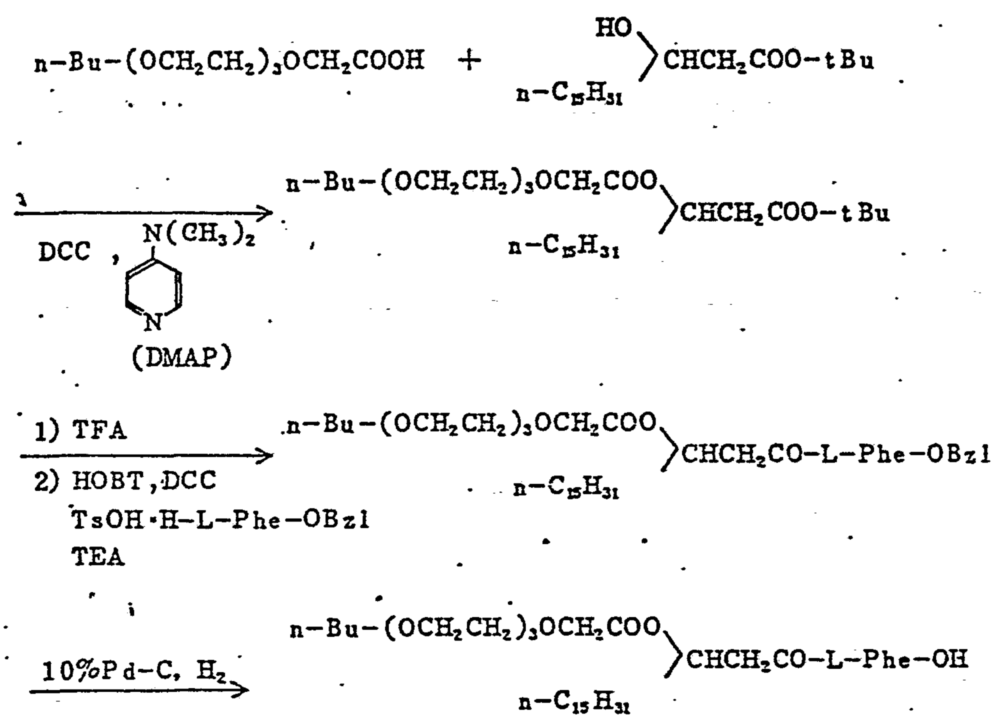


i) In 200 ml of methylenechloride were dissolved 1.32 g of 3,6,9,12-tetraoxahexadecanoic acid and 1.78 g of 3-hydroxyoctadecanoic acid t-butyl ester and after adding thereto 1.14 g of DCC and 61 mg of DMAP under ice-cooling, the mixture was stirred for 24 hours at room temperature. The reaction mixture was filtered and the filtrate was concentrated under reduced pressure. To the concentrate was added 50 ml of ethyl acetate and the solution formed was washed successively with cool 0.5 N hydrochloric acid, saturated saline solution, 4% aqueous sodium hydrogen

carbonate and saturated saline solution, dried over anhydrous sodium sulfate, and concentrated under reduced pressure. The residue thus obtained was subjected to column choromatography (silica gel: 50 g; benzene-ethyl acetate 5:1 v/v) to provide colorless liquid 1.2g of 3-(3,6,9,12-tetraoxahexadecanoyloxy)octadecanoic acid t-butyl ester.

IR(neat) 2910, 2840, 1755, 1730 $cm^{-1}$

NMR($CDCl_3$, δ) 0.92 (6H, m) 1.2～1.7 (32H, m) 1.43 (9H, s) 2.49 (2H, d, J=7Hz) 3.47 (2H, t, J=7Hz) 3.58～3.80 (12H, m) 4.12 (2H, s) 5.30 (1H, m)

ii) To 2.55 g of the compound obtained at 19-i) above was added dropwise 32 ml of TFA while cooling to -30°C. The mixture was stirred for 1 hour at room temperature. The reaction mixture was concentrated under reduced pressure and the residue obtained was dissolved in ether; the ether solution was washed with water, dried over anhydrous sodium sulfate, and distilled to remove the solvent. 2.24 g of a colorless oily material was obtained. 920 mg of the oily material was dissolved in 10 ml of methylenechloride and after adding thereto 230 mg of

HOBT and adding 350 mg of DCC under ice-cooling, the mixture was stirred for 1 hour at room temperature. To the mixture were added 727 mg of L-phenylalanine benzyl ester TsOH salt, and 238 μl of TEA under ice-cooling, and the mixture was stirred for 20 hours. The reaction mixture was filtered and the filtrate was washed successively with cool 0.5 N aquous hydrochloric acid, saturated saline solution, 4 % aquous sodium hydrogencarbonate and then saturated saline solution, and dried over anhydrous sodium sulfate, and then solvent was distilled away under reduced pressure to provide a residue. The residue thus obtained was subjected to column chromatography (silica gel: 30 g; chloroform:methanol 100:1 v/v) to provide 1.0 g colorless liquid N-[3-(3-, 6,9,12-tetraoxahexadecanoyloxy)octadecanoyl]-L-phenylalanine benzyl ester.

IR(neat) 3280, 2910, 2840, 1735, 1650 $cm^{-1}$

NMR($CDCl_3$, δ) 0.8 ~ 1.0 (6H, m) 1.1 ~ 1.7 (32H, m) 1.47 (2H, m) 3.10 (2H, d, J=7Hz) 3.46 (2H, t, J=7Hz) 3.60 ~ 3.75 (12H, m) 4.07 (2H, s) 4.90 (1H, m) 5.14 (2H, s) 5.22 (1H, m) 6.22 (1H, d, J=8Hz) 6.96 ~ 7.10 (2H, m) 7.15 ~ 7.45 (8H, m)

iii) In a mixture of 10 ml of methanol and 10 ml of ethyl acetate was dissolved 1 g of the compound obtained at 19-ii) above and the solution was hydrogenated over 100 mg of Pd-C under normal pressure at room temperature. The reaction mixture was filtered. The filtrate was concentrated under reduced pressure to provide 770 mg of white solid N-[3-(3,6,9,12-tetraoxahexadecanoyloxy)octadecanoyl]-L-phenylalnine.

IR(KBr) 3320, 2910, 2840, 1735, 1635 $cm^{-1}$

NMR($CDCl_3$, δ) 0.8～1.0 (6H, m) 1.1～1.7 (32H, m) 2.47 (2H, m) 3.16 (2H, m) 3.48 (2H, t, J=7Hz) 3.55～3.75 (12H, m) 4.07 (2H, s) 4.85 (1H, m) 5.23 (1H, m) 6.46 (1H, m) 7.24 (5H, m)

Example 20

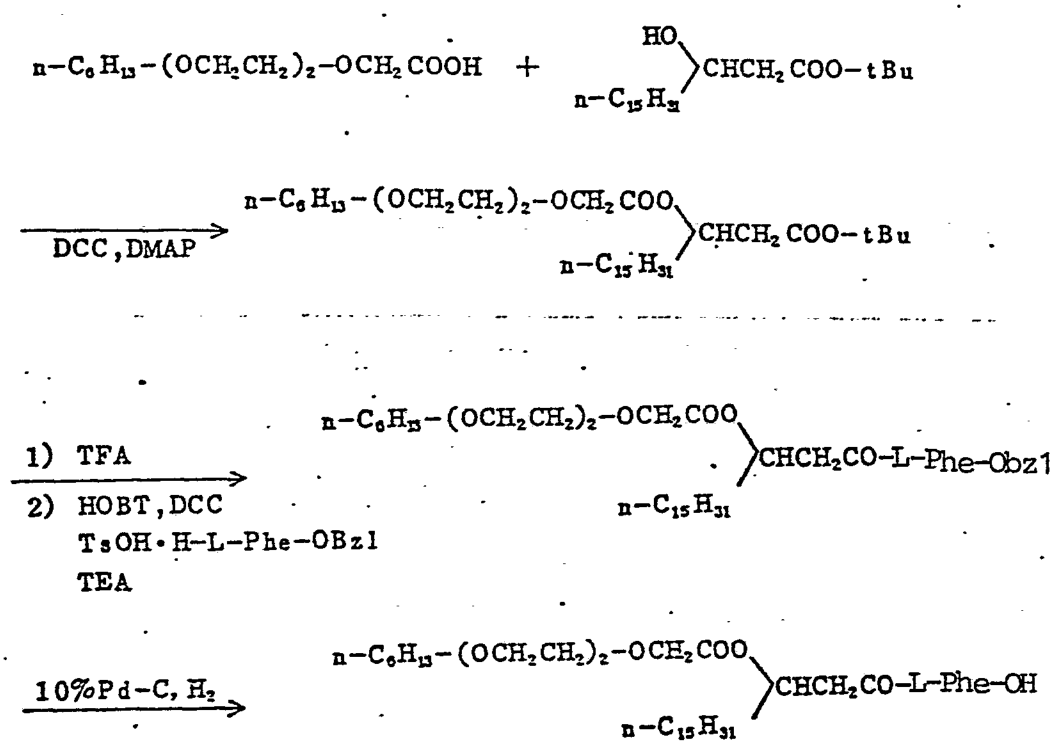


i) By following the same procedure as in Example 19-i) using 1.24 g of 3,6,9-trioxapentadecanoic acid and 1.78 g of 3-hydroxyoctadecanoic acid tert-butyl ester, 1.44 g of colorless liquid 3-(3,6,9-trioxapentadecanoyloxy)octadecanoic acid tert-butyl ester was obtained.

IR(neat) 2910, 2840, 1750, 1725 $cm^{-1}$

NMR($CDCl_3$, δ) 0.88(6H, m) 1.1～1.7(36H, m) 1.42(9H, s) 2.49(2H, d, J=7Hz) 3.45(2H, t, J=7Hz) 3.6～3.8(8H, m) 4.08(2H, s) 5.2(1H, m)

ii) By following the same procedure as in Example 19-ii) using 1.34 g of the compound obtained at 20-i) above, 1.23 g of colorless liquid N-[3-(3,6,9-trioxapentadecanoyloxy)octadecanoyl]-L-phenylalanine benzyl ester was obtained.

IR(neat) 3290, 2920, 2840, 1740, $1650cm^{-1}$

NMR($CDCl_3$, δ) 0.8～1.0(6H, m) 1.2～1.7(36H, m) 2.47(2H, m) 3.10(2H, d. J=7Hz) 3.45 (2H, t, J=7Hz) 3.55～3.75(8H, m) 4.08 (2H, s) 4.92(1H, m) 5.15(2H, m) 5.20(1H, m) 6.19(1H, d, J=9Hz) 6.95～7.15(2H, m) 7.16～7.45(8H, m)

iii) By following the same procedure as in Example 19-iii) using 1.2 g of the compound obtained at 20-ii) above, 910 mg of colorless solid N-[3-(3,6,9-trioxapentadecanoyloxy)octadecanoyl]-L-phenylalanine was obtained.

IR(KBr) 3320, 2910, 2840, 1730, 1635$cm^{-1}$

NMR($CDCl_3$, δ) 0.88(6H, m) 1.1~1.7(36H, m) 2.46(2H, m) 3.15(2H, m) 3.46(2H, t, J=7Hz) 3.55~3.70(8H, m) 4.06(2H, s) 4.83(1H, m) 5.22(1H, m) 6.39(1H, m) 7.1~7.4(5H, m) 7.7(1H, s)

Example 21

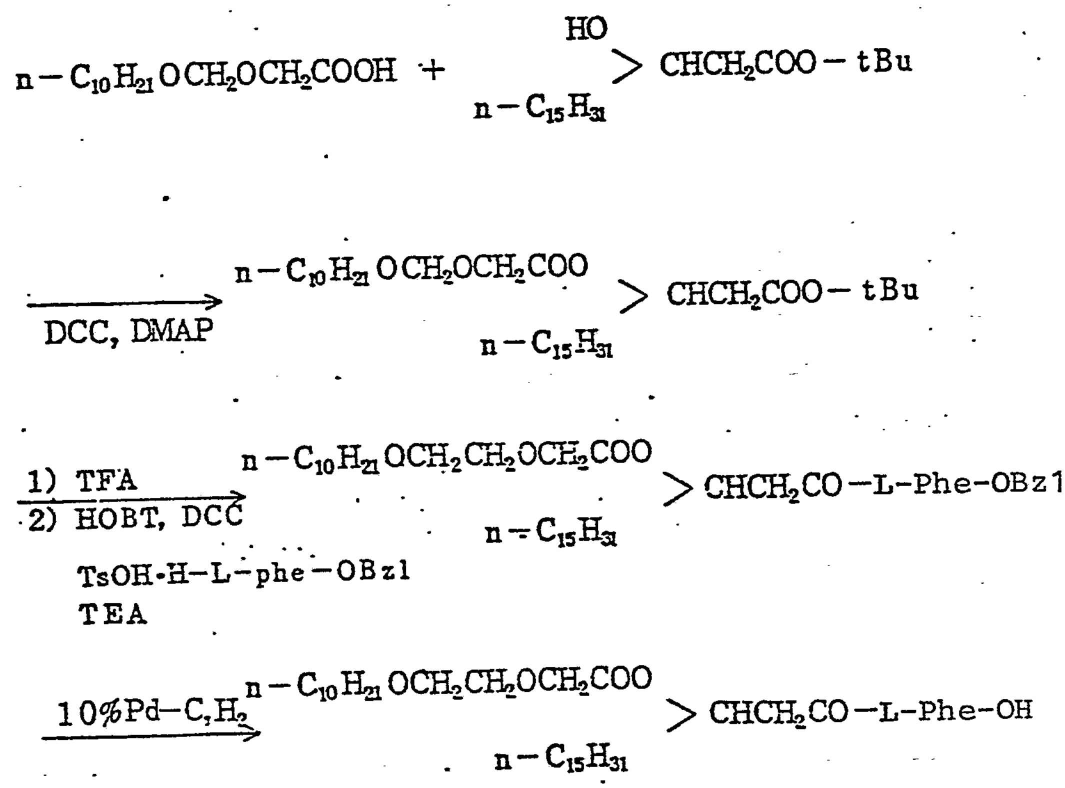


i) By following the same procedure as in Example 19-i) using 1.3 g of 3,6-dioxahexadecanoic acid and 1.78 g of 3-hydroxyoctadecanoic acid t-butyl ester, 1.5 g of colorless liquid 3-(3,6-dioxahexadecanoyloxy)octadecanoic acid tert-butyl ester was obtained.

IR (neat) 2910, 2840, 1755, 1730 $cm^{-1}$

NMR ($CDCl_3$, δ) 0.88 (6H, m) 1.1~1.7 (44H, m) 1.43 (9H, s) 2.50 (2H, d, J=7 Hz) 3.47 (2H, t, J=7 Hz) 3.67 (4H, m) 4.13 (2H, s) 5.11 (1H, m)

ii) By following the same procedure as in Example 19-ii) using 800 mg of the compound obtained at 21-i) above, colorless liquid 560 mg of N-[3-(3,6-dioxahexadecanoyloxy)octadecanoyl]-L-phenylalanine benzyl ester was obtained.

IR (neat) 3300, 2910, 2840, 1730, 1635 $cm^{-1}$

NMR ($CDCl_3$, δ) 0.88 (6H, m), 1.1~1.8 (44H, m) 2.46 (2H, m) 3.10 (2H, d, J=7 Hz), 3.45 (2H, t, J=7 Hz), 3.64 (4H, m) 4.07 (2H, s), 4.9 (1H, m) 5.15 (2H, s) 5.20 (1H, m) 6.12 (1H, d, J=9 Hz) 6.95~7.10 (2H, m) 7.2~7.4 (8H, m)

iii) By following the same procedure as in Example 19-iii) using 560 mg of the compound obtained at 21-ii) above, 390 mg of white N-[3-(3,6-dioxahexadecanoyloxy)hexadecanoyl]-L-phenylalanine was obtained.

IR(KBr) 3300, 2910, 2840, 1730, 1640 $cm^{-1}$

NMR ($CDCl_3$, δ) 0.88 (6H, m) 1.2～1.7 (44H, m) 2.47 (2H, m) 3.14 (2H, m) 3.49 (2H, t, J=7Hz) 3.66 (4H, s) 4.06 (2H, d, J=2Hz) 4.86 (1H, m) 5.20 (1H, m) 6.0～6.35 (2H, m) 7.26 (5H, m)

Example 22

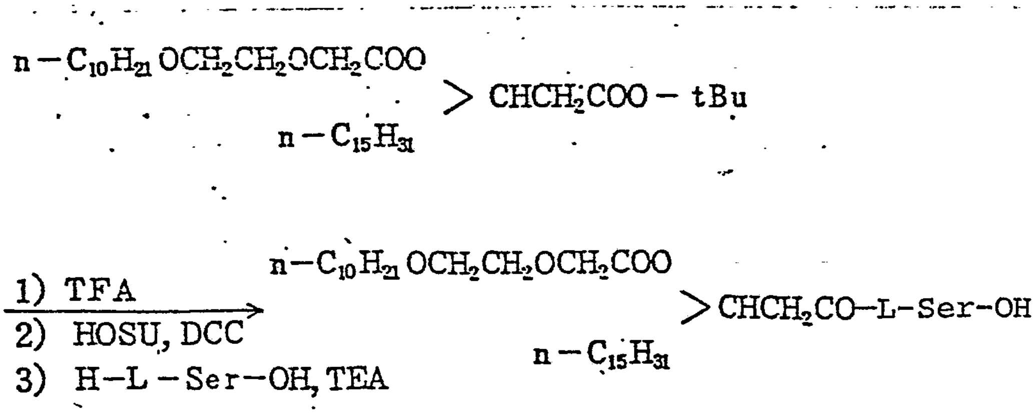

20 ml of TFA was added dropwise to 750 mg of the compound obtained by Example 21-i) under ice-cooling, and the resultant mixture was stirred for 2 hours at room temperature. The mixture was concentrated under reduced pressure. The formed residue was dissolved in 50 ml of ether, washed with water, and dried over anhydrous magnesium sulfate. The solvent was distilled away under reduced pressure to provide a colorless liquid. After adding thereto 138 mg of HOSU and 15 ml of dioxane, and adding thereto 249 mg of DCC under ice-cooling, the mixture was stirred for 4 hours at room temperature. The reaction mixture was filtered and the filtrate was concentrated under reduced pressure. The residue thus obtained was dissolved in 20 ml of DMF. The solution was cooled to 0°C and a solution of 127 mg of L-serine and 170 µl of TEA in 20 ml of water was added to the solution. The mixture was stirred for 2 days at room temperature. The reaction mixture was concentrated under reduced pressure, and 30 ml of water was added to the formed residue. The formed solution was acidified with 0.1 N hydrochloric acid, and extracted twice each time with 50 ml of chloroform. The chloroform layers were combined, washed twice with saline solution, dried over anhyrous sodium sulfate, and then concentrated under reduced pressure to provide a pasty residue. The residue obtained was subjected to column chromatography (silica gel:50 g; chloroform: methanol 100:1 v/v ), to provide 200 mg of white solid material of N-[3-(3,6-dioxahexadecanoyloxy)octadecanoyl]-L-serine.

IR(KBr) 3300, 2910, 2840, 1735, 1640 $cm^{-1}$

NMR ($CDCl_3$, δ) 0.8~1.0 (6H, m) 1.1~1.8 (44H, m) 2.58 (2H, m) 3.50 (2H, t, J=7Hz) 3.68 (4H, m) 4.0 (2H, m) 4.18 (2H, s) 4.65 (1H, m) 5.42 (2H, m) 7.05 (1H, m)

Example 23

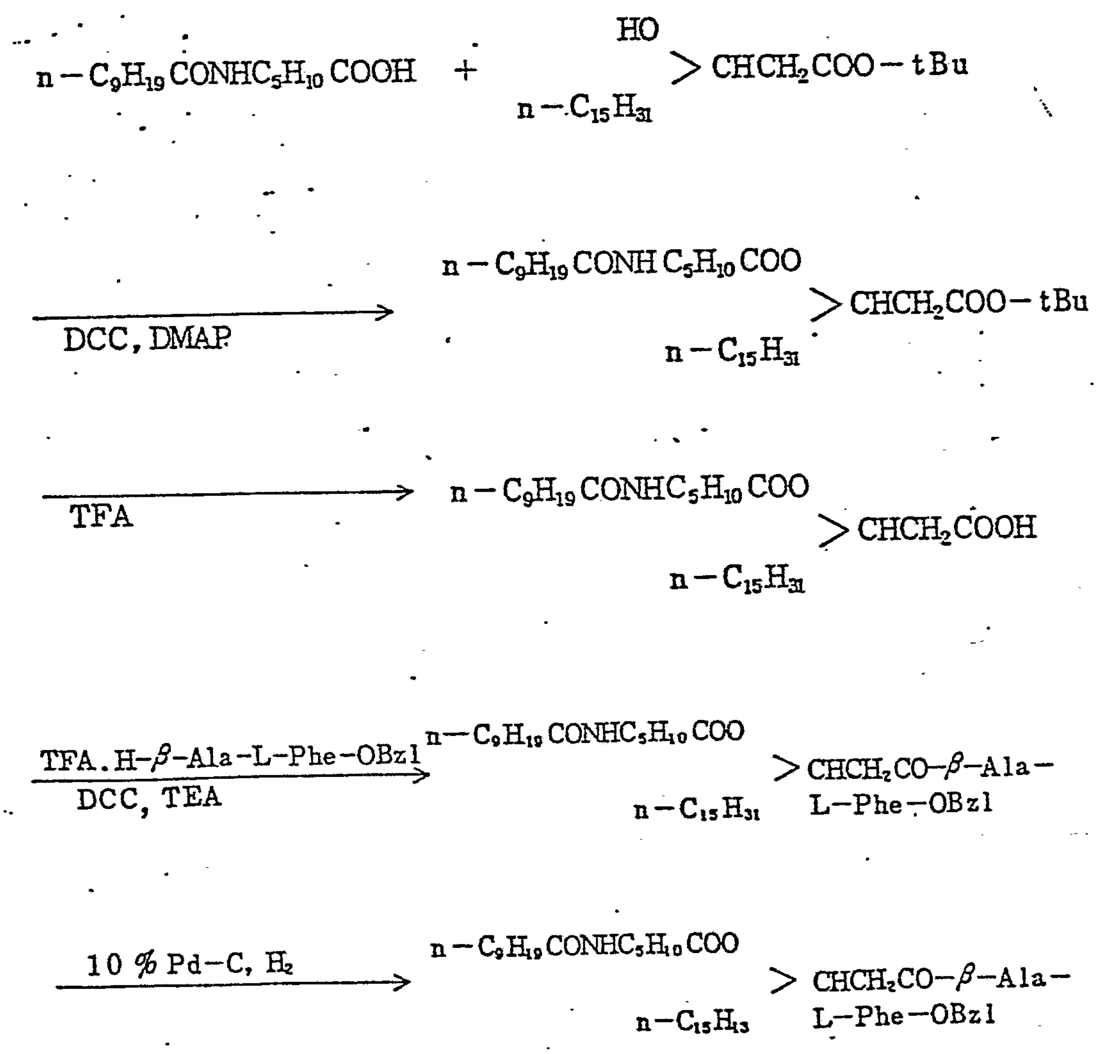

i) 1.57 g of 3-hydroxyoctadecanoic acid t-butyl ester and 1.30 g of 6-decanamidohexanoic acid were mixed with 30 ml of methylenechloride and after adding thereto 50 mg of DMAP and 930 mg of DCC while stirring, the mixture was stirred for 2 days at room temperature. Precipitates formed were removed by filtration and the filtrate was washed with 10 ml of 0.1 N hydrochloric acid, and then washed twice with water, dried over anhydrous magnesium sulfate, and concentrated. The concentrate was subjected to column chromatography and eluted with chloroform to provide 610 mg of 3-(6-decanamidohexanoyloxy)octadecanoic acid t-butyl ester.

ii) To 700 mg of the compound obtained at 23-i) above was added 8 ml of TFA , and the mixture was stirred for 2 hours at room temperature. TFA was distilled off under reduced pressure and after decantation with petroleum ether, the residue was subjected to column chromatography and eluted with methanol-chloroform (5:95 v/v) to provide 500 mg of 3-(6-decanamidohexanoyloxy)octadecanoic acid.

IR(KBr) 3300, 2900, 2830, 1720, 1695, 1620 $cm^{-1}$

NMR($CDCl_3$, $\delta$) 0.90 (6H, t, J=6Hz), 1.00~1.80 (48H, m) 2.08~2.40 (4H, m), 2.56 (2H, d, J=8Hz), 3.08~3.40 (2H, m), 5.10~5.46 (1H, m)

iii) 485 mg of the compound obtained at 23-ii) above was mixed with 10 ml of methylenechloride. 375 mg of β-alanyl-L-phenylalanine benzyl ester TFA salt and 0.11 ml of TEA and 175 mg of DCC were added succesively to the mixture under ice-cooling. After 30 minutes, the ice-bath was removed and the mixture was stirred overnight. Precipitates formed were removed by filtration and the filtrate was washed with water and dried over anhydrous magnesium sulfate. The solvent was distilled off under reduced pressure and the residue thus formed was subjected to silica gel column chromatography and eluted with methanol:chloroform (5:95 v/v) to provide 730 mg of N-[3-decanamidohexanoyloxy)octadecanoyl]-β-alanyl-L-phenylalanine benzyl ester.

iv) In a mixture of 30 ml of tetrahydrofuran and 10 ml of methanol was dissolved 705 mg of the compound obtained at 23-iii) above. The compound was hydrogenated over 80 mg of 10% Pd-C, and the catalyst was removed by filtration. The solvent was distilled off to provide 660 mg of N-[3-(6-decanamidohexanoyloxy)octadecanoyl]-β-alanyl-L-phenylalanine.

IR(KBr) 3290, 2910, 2840, 1720, 1635 $cm^{-1}$

NMR ($CDCl_3$, δ) 0.88 (6H, t, J=6Hz), 1.04~2.00 (48H, m), 2.00~2.52 (8H, m) 3.04~3.48 (6H, m), 4.64~4.92 (1H, m), 5.04~5.28 (1H, m), 7.24 (5H, s)

Example 24

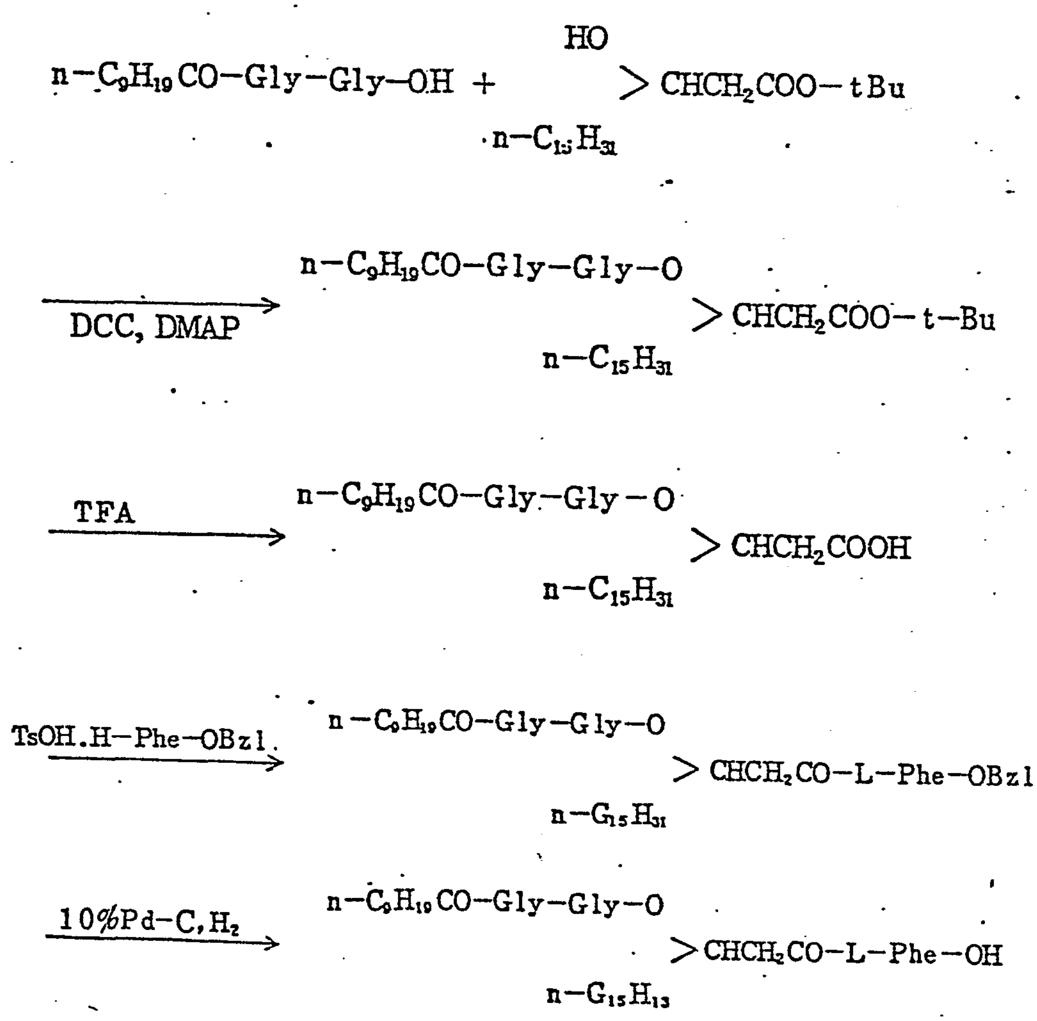

i) 1.96 g of 3-hydroxyoctadecanoic acid tert-butyl ester and 1.43 g of N-octanoylglycylglycine were mixed with 40 ml of methylenechloride. By following the same procedure as in Example 23-i) after adding 60 mg of DMAP and 1.14 g of DCC, 970 mg of 3-(N-octanoylglycylglycyloxy)-octadecanoic acid tert-butyl ester was obtained.

IR(KBr) 3280, 2920, 2840, 1730, 1660, 1630 $cm^{-1}$

NMR($CDCl_3$, δ) 0.88 (6H, t, J=6 Hz), 1.04~2.00 (51H, m), 2.10~2.40 (2H, m), 2.50 (2H, d, J=8 Hz), 3.88~4.28 (4H, m), 5.12~5.40 (1H, m)

ii) By treating 770 mg of the ester compound obtained at 24-i) above as in Example 23-ii) (using 8 ml of TEA), a carboxylic acid (free compound) was formed. After drying the product, 2 ml of methylenechloride was added thereto, and 530 mg of L-phenylalanine benzyl ester p-toluenesulfonic acid salt was mixed with the product. To the mixture were added 0.17 ml of TEA and 255 mg of DCC under ice-cooling while stirring. The formed mixture was stirred overnight at room temperature. Precipitate thus formed was removed by filtration, and the filtrate was washed with water, dried and subjected to silica gel column chromatography and eluted with methanol -chloroform (5:95 v/v) to provide 316 mg of N-[3-(N-decanoylglycylglycyloxy)octadecanoyl]-L-phenylalanine benzyl ester.

iii)

300 mg of the ester compound formed as at 24-ii) above was dissolved in a mixture of 20 ml of tetrahydrofuran and 10 ml of methanol. By following the same procedure as in Example 23-iv), 270 mg of N-[3-(N-decanoylglycylglycyloxy)octadecanoyl]-L-phenylalanine was obtained.

IR(KBr) 3300, 2900, 2840, 1740, 1650, 1630 $cm^{-1}$

NMR ($CDCl_3$, δ) 0.88 (6H, t, J=6 Hz), 1.08 ~ 1.92 (42H, m), 2.08 ~ 2.40 (2H, m), 2.40 ~ 2.56 (2H, d, J=8 Hz), 3.04 ~ 3.24 (2H, m), 3.80 ~ 4.20 (4H, broad), 4.72 ~ 5.00 (1H, m), 5.12 ~ 5.48 (1H, m), 7.28 (5H, s)

Example 25

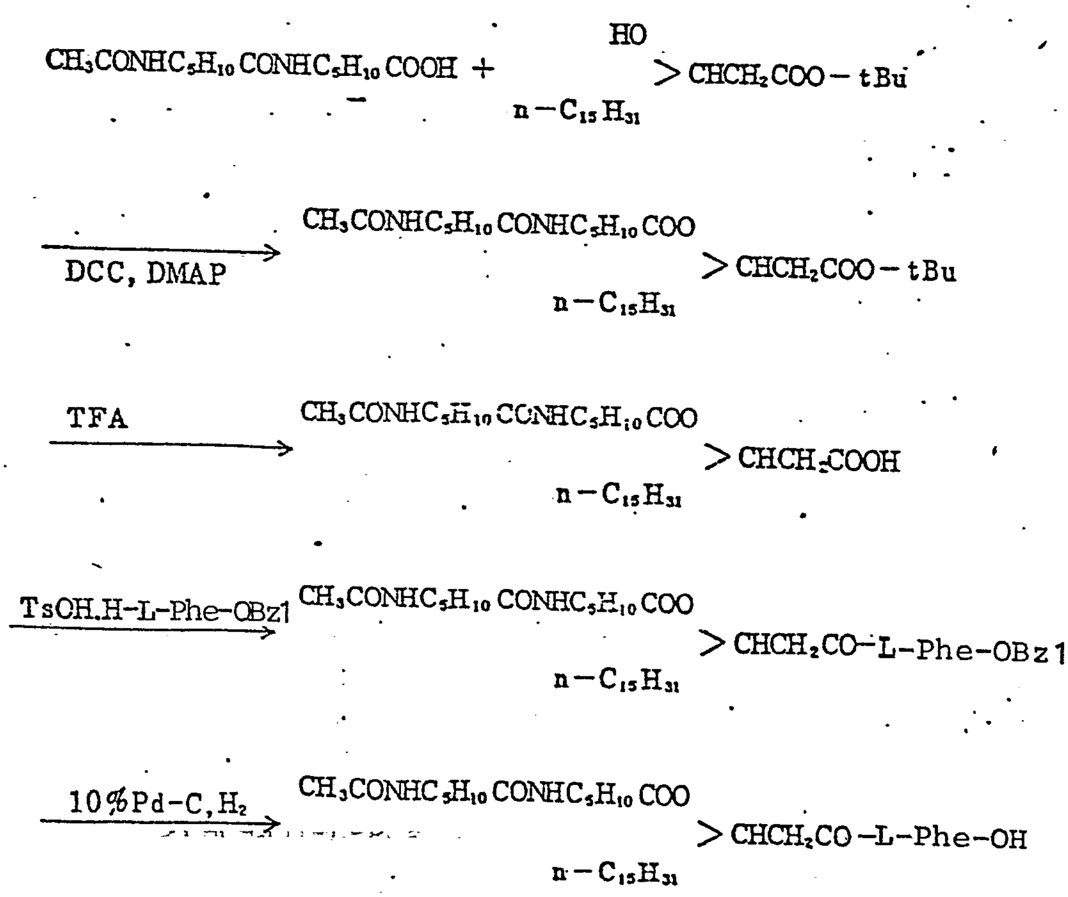

i) 2.35 g of 3-hydroxyoctadecanoic acid t-butyl ester and 1.80 g of 6-(6-acetamidohexanamido)hexanoic acid were mingled in 40 ml of methylenechloride and after adding thereto 80 mg of DMAP and 1.36 g of DCC, the mixture was treated in the same manner as in Example 23-i) to provide 1.82 g of 3-[6-(6-acetamidohexanamido)hexanoyloxy]octadecanoic acit t-butyl ester.

IR(KBr)3280, 3070, 2920, 2850, 1730, 1660 $cm^{-1}$

NMR ($CDCl_3$, δ) 0.92 (3H, t, J=6Hz), 1.08 ~ 1.80 (49H, m), 1.96 (3H, s), 2.04 ~ 2.52 (6H, m), 3.00 ~ 3.36 (4H, m), 5.04 ~ 5.28 (1H, m)

ii) 1.80 g of the above 25-i) ester compound was treated with 8 ml of TFA to convert the ester to free carboxylic acid and after adding thereto 5 ml of methylenechloride, 1.20 g of L-phenylalanine benzyl ester TsOH salt, 0.4 ml of TEA and 590 mg of DCC, the mixture was trated in the same manner as in Example 14-ii) to provide 740 mg of N-[3-[6-(6-acetamidohexanamido)hexanoyloxy]octadecanoyl]-L-phenylalanine benzyl ester. 660 mg of the ester compound thus

were dissolved in a mixture of 30 ml of tetrahydrofuran and 15 ml of methanol and after carrying out catalytic reduction using 100 mg of 10% Pd-C and treating in the same manner as in Example 23-iv), 450 mg of N-[3-[6-(6-acetamidohexanamido)hexanoyloxy]octadecanoyl]-L-phenylalanine was obtained.

IR(KBr) 3280, 3070, 2920, 2850, 1730, 1650, 1630 $cm^{-1}$

NMR ($CDCl_3$, δ) 0.96 (3H, t, J=6Hz), 1.08 ~ 1.92 (40H, m), 2.00 (3H, s), 2.08~2.48 (6H, m), 3.04~3.48 (6H, m) 4.76~4.92 (1H, m), 5.08~5.20 (1H, m), 7.24 (5H, s)

Example 26

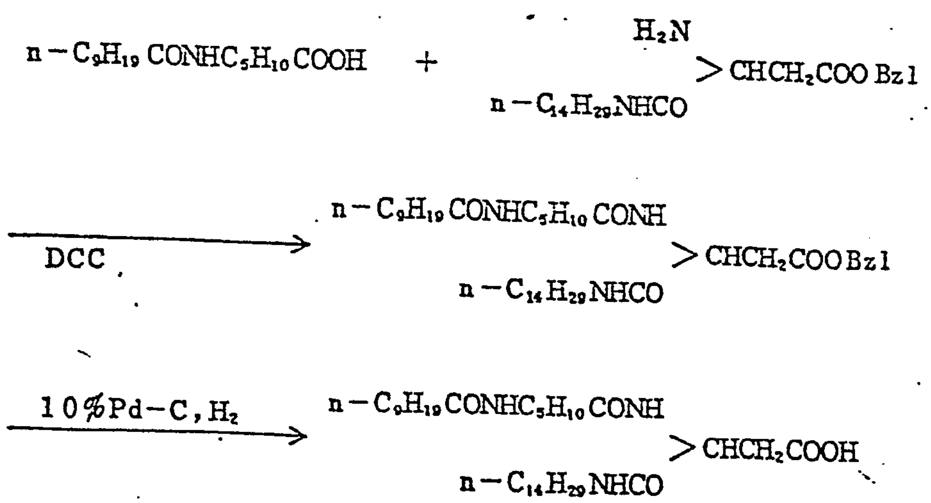

820 mg of (S)-3-amino-3-tetradecylcarbamoylpropionic acid benzyl ester and 520 mg of 6-decanamidohexanoic acid were mingled in 10 ml of methylenechloride and after adding thereto 400 mg of DCC while stirring under ice-cooling and removing the ice-bath 30 minutes thereafter, the mixture was stirred overnight.

Precipitates thus formed were removed by filtration, the filtrate was washed with water, dried over anhydrous magnesium sulfate, concentrated, and subjected to silica gel column chromatography. The product was eluted with ethyl acetate-chloroform (1:9 -- 1:1 v/v) to provide 600 mg of (S)-3-(6-decanamidohexanamido)-3-tetradecylcarbamoylpropionic acid benzyl ester. 1.00 g of the ester compound obtained was dissolved in a mixture of 30 ml of tetrahydrofuran and 10 ml of methanol and after carrying out catalytic reduction using 100 mg of 10% Pd-C and removing the catalyst by filtration, the solvent was distilled off to provide 670 mg of (S)-3-(6-decanamidohexanamido)-3-tetradecylcarbamoylpropionic acid.

IR(KBr)3300, 2900, 2840, 1720, 1690, 1630 $cm^{-1}$

NMR ($CDCl_3$+$CD_3OD$, δ) 0.88 (6H, t, J=6Hz), 1.04~1.88 (44H, m), 2.04~2.40 (4H, m), 2.72 (2H, d, J=8Hz), 3.04~3.36 (4H, m), 4.60~4.84 (1H, m)

Example 27

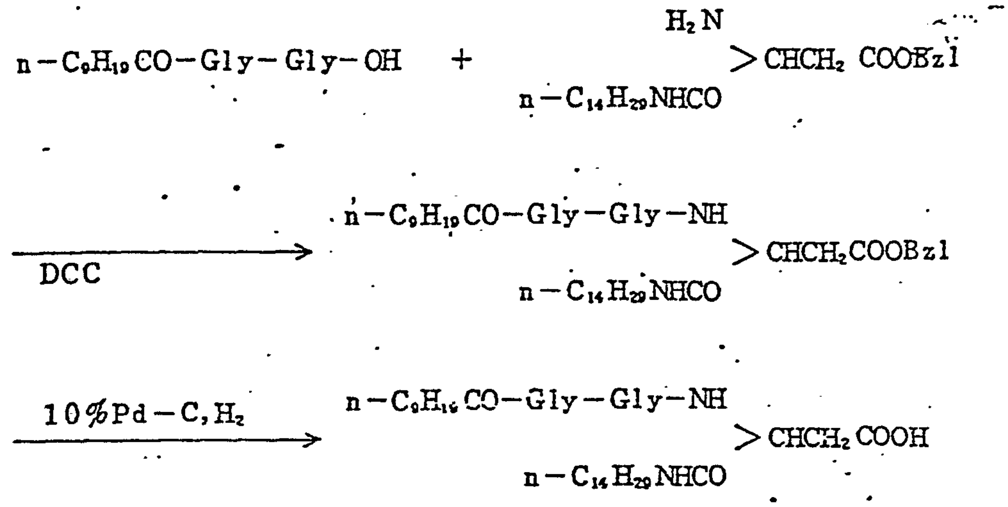


780 mg of (S)-3-amino-3-tetradecylcarbamoylpropionic acid benzyl ester and 490 mg of N-decanoylglycylglycine were mingled in 10 ml of DMF and after adding thereto 380 mg of DCC, the mixture was treated in the same manner as in Example 26. The product was eluted with ethyl acetate-chloroform-methanol (5:5:1 v/v) to provide 170 mg of (S)-3-(N-decanoylglycylglycylamido)-3-tetradecylcarbamoylpropionic acid benzyl ester.

380 mg of the ester compound thus obtained was dissolved in a mixture of 20 ml of tetrahydrofuran and 10 ml of methanol and after treating the mixture as in Example 26, 320 mg of (S)-3-(N-decanolylglycylglycylamido)-3-tetradecylcarbamoylpropionic acid was obtained.

IR (KBr) 3280, 3050, 2900, 2830, 1705, 1650 $cm^{-1}$

NMR ($CDCl_3 + CD_3OD$, δ) 0.88 (6H, t, J=6 Hz), 1.08~2.00 (38H, m), 2.08~2.44 (2H, m), 2.84 (2H, d, J=8 Hz), 3.04~3.36 (2H, m), 3.84~4.12 (4H, broad), 4.64~4.88 (1H, m)

Example 28

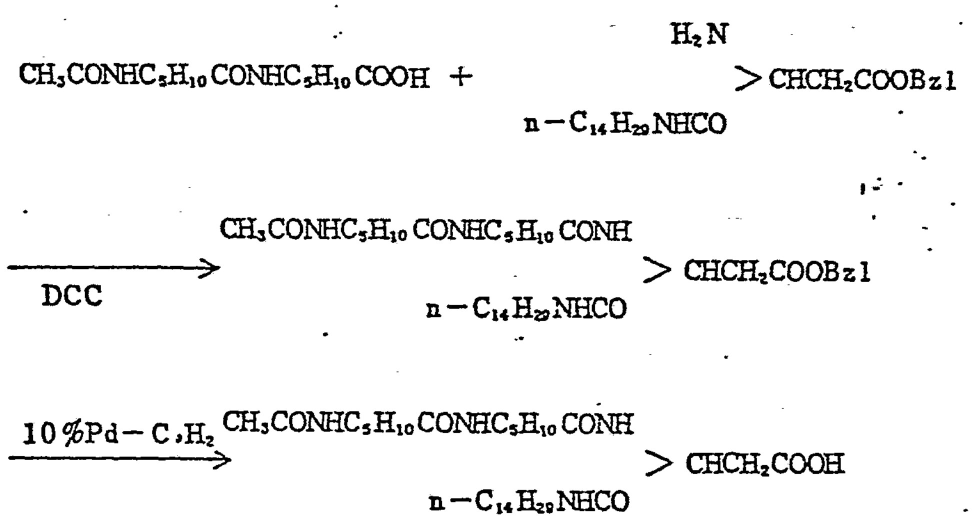


2.1 g of (S)-3-amino-3-tetradecylcarbamoylpropionic acid benzyl ester and 1.5 g of 6-(6-acetamidohexanamido)-hexanoic acid were mingled in 10 ml of methylene chloride and after adding thereto 1.1 g of DCC and treating the mixture as in Example 26, the product was subjected to silica gel column chromatography, and was eluted with methanol-chloroform (1:3 v/v) to provide 250 mg of (s)-3-[6-(6-acetamidohexanamido)-hexanamido]-3-tetradecylcarbamoylpropionic acid benzyl ester.

250 mg of the ester compound thus obtained was dissolved in a mixture of 10 ml of tetrahydrofuran and 15 ml of methanol and after treating the mixture

as in Example 26, 210 mg of (S)-3-[6-(6-acetamidohexanamido)hexanamido]-3-tetradecylcarbamoylpropionic acid was obtained.

(KBr) 3270, 3070, 2910, 2840, 1720, 1630$cm^{-1}$

NMR ($CDCl_3$ + $CD_3OD$, $\delta$) 0.88 (3H, t, J=6Hz), 1.04~1.96 (36H, m) 2.00 (3H, s), 2.08~2.48 (4H, m), 2.50~2.86 (2H, m), 3.08~3.40 (6H, m) 4.52~4.80 (1H, m)

Example 29

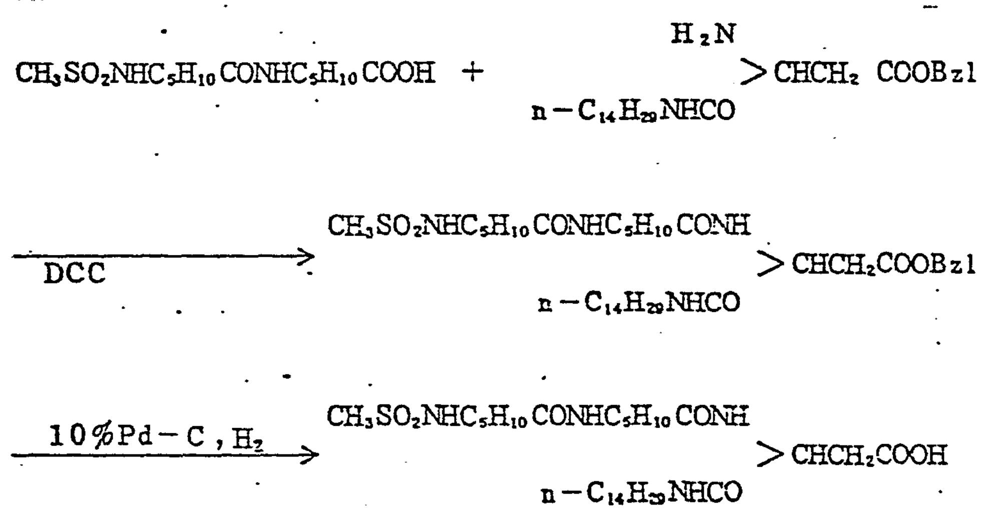


1.05 g of (S)-3-amino-3-tetradecylcarbamoylpropionic acid benzyl ester and 1.00 g of 6-(6-methanesulfonamidohexanamido)hexanoic acid were mingled in 20 ml of methylenechloride and after adding thereto 500 mg of DCC and treating the mixure as in Example 26, 140 mg of (S)-3-[6-(6-methanesulfonamidohexanamido)hexanamido]-3-tetradecylcarbamoylpropionic acid benzyl ester was obtained.

35 mg of the ester compound thus obtained was dissolved in 10 ml of methanol and after treating the mixture as in Example 26, 110 mg of (S)-3-[6-(6-methanesulfonamidohexanamido)hexanamido]-3-tetradecylcarbamoylpropionic acid was obtained.

IR(KBr) 3280, 2920, 2850, 1730, 1640 $cm^{-1}$

NMR ($CDCl_3$ + $CD_3OD$, δ) 0.92 (3H, t, J=6 Hz), 1.08~2.00 (36H, m) 2.04~2.50 (4H, m) 2.50~2.80 (2H, m), 2.96 (3H, s), 3.04~3.40 (6H, m), 4.60~4.80 (1H, m)

C L A I M S :

1. A fatty acid derivative represented by following general formula (I), or a salt thereof

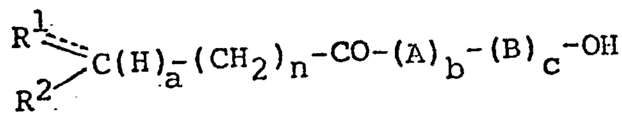

(I)

in which

$R^1$ represents an alkanoylamino group the carbon chain of which may be interrupted by at least one oxygen atom, an alkanoyloxy group the carbon chain of which is interrupted by at least one oxygen atom, an alkoxyimino group, or a group shown by the formula $R^3-(NH-Y-CO)_m-Z-$ (wherein $R^3$ represents an alkanoyl group or a $C_1-C_5$ alkylsulfonyl group; Y represents an alkylene group; m represents 1 or 2; and Z represents an oxygen atom or an imino group);

$R^2$ represents an alkyl group, an alkylaminocarbonyl group the carbon chain of which may be interrupted by at least one oxygen atom, or an alkoxyaminocarbonyl group the carbon chain of which is interrupted by at least one oxygen atom;

$\equiv$ represents a single bond or a double bond;

a is zero when $\equiv$ is a double bond and 1 when $\equiv$ is a single bond;

b is 0 or 1; c is 0 or 1;

n represents an integer of 1 to 3; and A and B are the same or different amino acid residues.

2. A compound according to claim 1 which is represented by the following general formula (I'), or a salt thereof

$$\begin{matrix}R^{1'}\\ R^{2'}\end{matrix}\!\!>CH-(CH_2)_n-CO-(A')_b-(B')_c-OH \qquad (I')$$

in which b is 0 or 1; c is 0 or 1

$R^{1'}$ represents a group shown by the formula $R^{10}-(O-Y^1)_p-O-Y^2-D^1$ (wherein $R^{10}$ represents an alkyl group having 1 to 12 carbon atoms; $Y^1$ represents an alkylene group;

p represents 0 or an integer of 1 to 3;

$Y^2$ represents a single bond or a alkylene group and $D^1$ represents a carbamide group or a carbonyloxy group);

$R^{2'}$ represents an alkyl group having 1 to 20 carbon atoms or a group shown by the formula $R^{10}-(O-Y^1)_p-O-Y^2-D^2-$ (wherein $R^{10}$, $Y^1$, $Y^2$ and p are as defined above and $D^2$ represents a carbamoyl group (-NHCO-));

n represents an integer of 1 to 3; and

A' and B', which may be the same or different, each represents a serine residue, a phenylalanine residue, a ß-alanine residue, a glutamic acid residue, an aspartic acid residue, a glycine residue, or a tyrosine residue.

3. One or more compounds according to claim 1 selected from

N-[3-(3,6-Dioxahexanoyloxy)octadecanoyl]-L-phenylalanine;

N-[3-(3,6-Dioxahexanoyloxy)octadecanoyl]-L-serine;

N-[3-(3,6,9-Trioxapentadecanoyloxy)octadecanoyl]-L-phenylalanine ;

N-[3-(3,6,9,12-Tetraoxahexadecanoyloxy)octadecanoyl]-L-phenylalanine ;

(S)-3-(3,6-Dioxahexadecanamido)-3-(3-oxatridecylcarbamoyl)propionic acid; and salts thereof.

4. A process of producing a fatty acid derivative according to claim 1 which comprises releasing any existing protective group from a compound represented by the formula

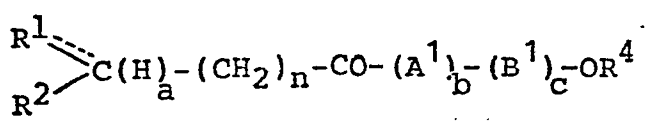

wherein $R^1$, $R^2$, ==== , a , b, c and n are as defined above; $A^1$ and $B^1$ represent amino acid residues which may have one or more protective groups; and $R^4$ represents a protective group for the carboxy group.

5. A process of producing a fatty acid derivative according to claim 1 which comprises reacting a

carboxylic acid represented by the general formula or a reactive carboxy derivative thereof

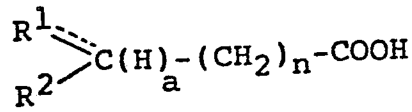

with an amino acid or a peptide represented by the general formula

$$H - (A^2)_b - B^2 - OR^5$$

wherein $R^1$, $R^2$, $\overline{\underline{----}}$, $(H)_a$ and n are as defined above; $A^2$ and $B^2$ represent amino acid residues which may have one or more protective groups; b is 0 or 1; and $R^5$ represents a hydrogen atom or a protective group for the carboxy group and releasing any protective group(s).

6. A process of producing a fatty acid derivative represented by the general formula

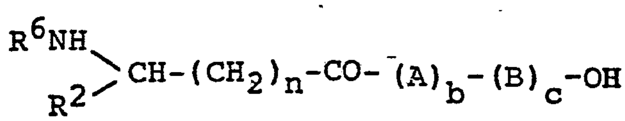

in which $R^2$, A, B, b, c and n are as defined above; and in which

$R^6$ represents an alkanoyl group the carbon chain of which may be interrupted by at least one oxygen atom or a group shown by the formula $R^3-(NH-Y-CO)_m-$ (where $R^3$ represents an alkanoyl group or a $C_1-C_5$ alkylsulfonyl group; Y represents an alkylene group; and m represents 1 or 2),

which comprises reacting an amine represented by the general formula

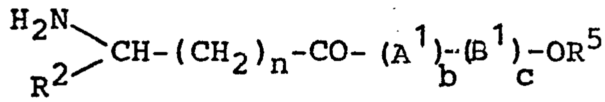

wherein $A^1$ and $B^1$ represent amino acid residues which may have one or more protective groups and $R^5$ represents a hydrogen atom or a protective group for the carboxy group with a carboxylic acid represented by the general formula

$$R^6 - OH$$

or a reactive derivative thereof and releasing any protective group(s).

7. A process of producing a fatty acid derivative represented by the general formula

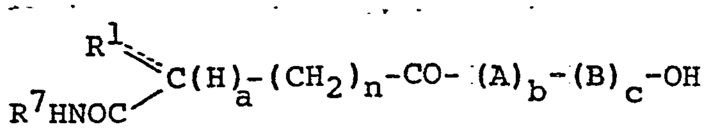

in which $R^1$, A, B, a, b, c and n are as defined above; and

$R^7$ represents an alkyl group the carbon chain of which may be interrupted by at least one oxygen atom or an alkoxy group the carbon chain of which is interrupted by at least one oxygen atom,

which comprises reacting a carboxylic acid represented by the general formula or a reactive derivative thereof

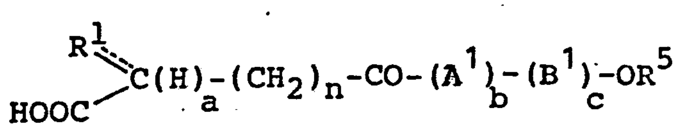

wherein $R^1$, a, ----, b, c and n are as defined above; $A^1$ and $B^1$ represent amino acid residues which may have one or more protective groups; and $R^5$ represents a hydrogen atom or a protective group for the carboxy group

with an amine represented by the general formula

$$R^7 - NH_2$$

and releasing any protective group(s).

8. A process of producing a fatty acid derivative represented by the general formula

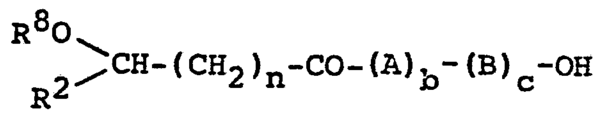

in which $R^2$, A, B, b, c and n are as defined above; and

$R^8$ represents an alkanoyl group the carbon chain of which is interrupted by at least one oxygen atom or a group shown by the formula $R^3-(NH-Y-CO)_m$ (wherein $R^3$ represents an alkanoyl group or a $C_1-C_5$ alkylsulfonyl group; Y represents an alkylene group; and m represents 1 or 2);

which comprises reacting an alcohol represented by the general formula,or an activated derivative thereof

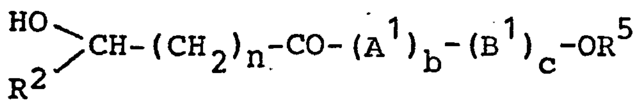

wherein $A^1$ and $B^1$ represent amino acid residues which may have one or more protective group; and $R^5$ represents a hydrogen atom or a protective group for the carboxy group with a carboxylic acid represented by the general formula

$$R^8 - OH$$

or a reactive derivative and releasing any protective group(s).

9. A process of producing a fatty acid derivative represented by the formula

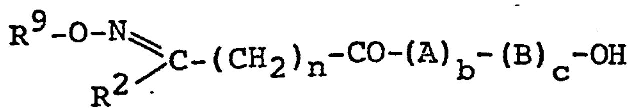

in which $R^2$, A, B, b, c and n are as defined above: and

$R^9$ represents an alkyl group,

which comprises reacting a substituted hydroxylamine represented by the general formula

$$R^9 - O - NH_2$$

0136879

with a ketocarboxylic acid represented by the general formula

$$R^2-CO-(CH_2)_n-CO-(A^1)_b-(B^1)_c-OR^4$$

wherein $A^1$ and $B^1$ represent amino acid residues which may have one or more protective groups; and $R^4$ represents a protective group for the carboxy group, and then releasing any protective group(s).

10. A medicament preparation composition containing an effective amount of a compound according to any of claims 1 to 3.